# EUROPEAN PATENT APPLICATION

(11) **EP 4 276 455 A1**
(43) Date of publication of application: **15.11.2023**
(21) Application number: 21917669.0
(22) Date of filing: 20.12.2021
(51) Int. Cl.: G01N 27/414

(54) **TRANSISTOR-TYPE POLYAMINE SENSOR**

(30) Priority: 08.01.2021 JP 2021001818
(71) Applicant: The University of Tokyo, Bunkyo-ku, Tokyo 113-8654 (JP); TOYOBO CO., LTD., Osaka-shi Osaka 5300001 (JP)
(72) Inventor: MINAMI, Tsuyoshi, Tokyo 113-8654 (JP); ASANO, Koichiro, Tokyo 113-8654 (JP); TANAKA, Hikaru, Otsu-shi, Shiga 520-0292 (JP); HIRATA, Akari, Otsu-shi, Shiga 520-0292 (JP); HAGIYA, Kazutake, Otsu-shi, Shiga 520-0292 (JP)
(74) Representative: Müller-Boré & Partner Patentanwälte PartG mbB
(86) International application number: PCT/JP2021/046993
(87) International publication number: WO 2022/149449

(57) **Abstract**

The present invention provides a transistor-based polyamine sensor with which it is possible to detect a polyamine easily and with high sensitivity. Specifically, the present invention provides a transistor-based polyamine sensor comprising a detection site, wherein an organic compound containing a carboxy group and a thiol group is immobilized on all or part of the surface of the detection site via the thiol group of the organic compound, a heavy metal ion is bound to the carboxy group of the organic compound, and a change in threshold voltage caused by bonding of the heavy metal ion to a polyamine is measured to detect the polyamined.

## Description

### Technical Field

The present invention relates to a transistor-based polyamine sensor.

### Background Art

"Polyamine" is a general term for compounds that have two or more amino groups. Polyamines are present in *E. coli* and animal cells in the order of several mM to several tens of mM. Examples of polyamines include spermidine, spermine, etc.

Recently, polyamines have been reported to be involved in bacterial biofilm formation. Biofilm refers to a pellicle assembled from bacteria, which becomes, for example, slime in drains and increases the bacterial survival rate. Once a bacterial biofilm is formed, the bacteria have resistance to various antibiotics. Therefore, great importance is placed on quantitative analysis of polyamines involved in biofilm formation.

For example, Patent Literature (PTL 1) discloses a method for analyzing polyamines by high-performance liquid chromatography (HPLC).

Patent Literature (PTL 2) discloses a method for analyzing the amount of polyamine, comprising mixing a polyamine with succinimidyl pyrene butyrate in tetrahydrofuran-DMSO-water at room temperature to label the polyamine, and analyzing the quantity of polyamine based on the measurement of fluorescence intensity and/or fluorescence intensity decay curve of fluorescence emitted in a wavelength region of 450 to 520 nm.

### Citation List

### Patent Literature

PTL 1: JPS63-18265A
PTL 2: JPH10-142228A

### Summary of Invention

### Technical Problem

However, the HPLC method disclosed in PTL 1, which requires large equipment, cannot be considered to be a method for detecting polyamine easily. Further, the method of measuring the fluorescence intensity disclosed in PTL 2 comprises a complicated operation and thus has a problem in that it is difficult to detect polyamine in a solution easily and with high selectivity.

Accordingly, there is a demand for a means for detecting polyamine easily and with high sensitivity.

The present invention was made to solve the above problem. An object of the present invention is to provide a transistor-based polyamine sensor capable of detecting a polyamine easily and with high sensitivity.

### Solution to Problem

The present inventors conducted extensive research to achieve the above object. As a result, the inventors have found that the object can be achieved by a transistor-based sensor in which an organic compound containing a carboxy group and a thiol group is immobilized on the surface of a detection site of the sensor. The present invention was accomplished through further research based on the above finding by the present inventors.

Specifically, the present invention provides the invention according to the following embodiments.
Item 1. A transistor-based polyamine sensor comprising a detection site, wherein
   an organic compound containing a carboxy group and a thiol group is immobilized on all or part of the surface of the detection site via the thiol group of the organic compound;
   a heavy metal ion is bound to the carboxy group of the organic compound; and
   a change in threshold voltage caused by bonding of the heavy metal ion to a polyamine is measured to detect the polyamine.
Item 2. The transistor-based polyamine sensor according to Item 1, wherein the organic compound has a monocyclic heterocycle containing at least one member selected from the group consisting of a nitrogen atom, a sulfur atom, and an oxygen atom.
Item 3. The transistor-based polyamine sensor according to Item 1 or 2, wherein the organic compound is an organic compound represented by the following formula (1): 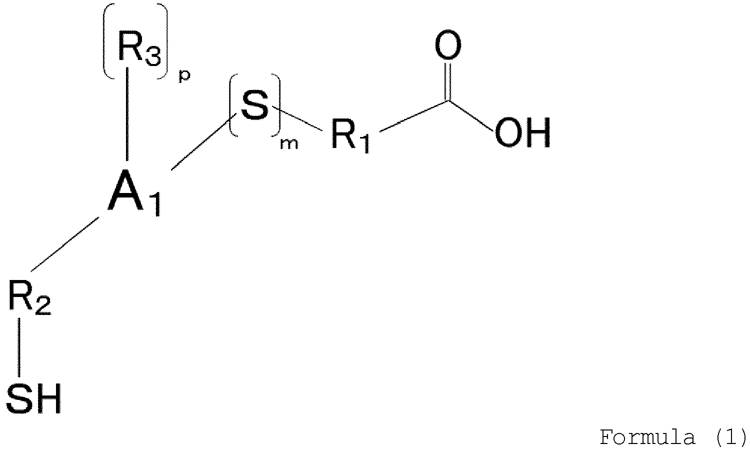 wherein
   A₁ represents an aromatic hydrocarbon group, an aliphatic hydrocarbon group having 1 to 10 carbon atoms, or a monocyclic heterocyclic group containing at least one member selected from the group consisting of a nitrogen atom, a sulfur atom, and an oxygen atom;
   R₁ represents an optionally substituted alkylene group having 1 to 10 carbon atoms,
   R₂ represents a single bond or an optionally substituted alkylene group having 1 to 10 carbon atoms;
   p is 0 or 1;
   when p is 1, R₃ represents a hydrogen atom or an alkyl group having 1 to 10 carbon atoms; and
   m is 0 or 1.
Item 4. The transistor-based polyamine sensor according to Item 3, wherein the monocyclic heterocyclic group represented by A₁ in the formula (1) is a 5-membered or 6-membered monocyclic heterocyclic group containing 1 or 2 nitrogen atoms.
Item 5. The transistor-based polyamine sensor according to Item 3 or 4, wherein the monocyclic heterocyclic group represented by A₁ in the formula (1) is a 5-membered monocyclic aromatic heterocyclic group containing 2 nitrogen atoms.
Item 6. The transistor-based polyamine sensor according to any one of Items 3 to 5, wherein the monocyclic heterocyclic group represented by A₁ in the formula (1) is thiadiazole.
Item 7. The transistor-based polyamine sensor according to any one of Items 3 to 6, wherein R₃ in the formula (1) is a hydrogen atom.
Item 8. The transistor-based polyamine sensor according to any one of Items 1 to 7, wherein the polyamine is at least one member selected from the group consisting of ethylenediamine, spermine, spermidine, 1,6-diaminohexane, cadaverine, putrescine, trimethylenediamine, histamine, and tryptamine.
Item 9. The transistor-based polyamine sensor according to any one of Items 1 to 8, wherein the heavy metal ion is at least one member selected from the group consisting of a divalent iron ion, a divalent cobalt ion, a divalent zinc ion, a divalent cadmium ion, a divalent lead ion, a divalent copper ion, and a divalent nickel ion.
Item 10. The transistor-based polyamine sensor according to any one of Items 1 to 9, wherein the transistor is an organic thin-film transistor.
Item 11. A polyamine detection method comprising bringing the transistor-based polyamine sensor of any one of Items 1 to 10 into contact with a sample containing a polyamine.
Item 12. Use of an organic compound containing a carboxy group and a thiol group as a transistor-based polyamine sensor.
Item 13. A method for identifying a polyamine, comprising
   creating a model that has undergone pattern learning by linear discriminant analysis, based on data on a change in transmission characteristics of a transistor-based polyamine sensor, the data being obtained by using the polyamine detection method of Item 11; and
   identifying the polyamine by pattern recognition.
Item 14. A method for measuring the concentration of a polyamine, comprising
   measuring the concentration of a polyamine to be measured in the presence of an impurity other than the polyamine to be measured, by using a support vector machine, based on data on a change in transmission characteristics of a transistor-based polyamine sensor, the data being obtained by using the polyamine detection method of Item 11.
Item 15. A transistor-based polyamine sensor comprising a detection site, wherein
   an organic compound containing a carboxy group and a thiol group is immobilized on all or part of the surface of the detection site via the thiol group of the organic compound;
   a heavy metal ion is bound to the carboxy group of the organic compound; and
   a change in threshold voltage caused by bonding of the heavy metal ion to a polyamine is measured to detect the polyamine.
Item 16. The transistor-based polyamine sensor according to Item 15, wherein the organic compound has a monocyclic heterocycle containing at least one member selected from the group consisting of a nitrogen atom, a sulfur atom, and an oxygen atom.
Item 17. The transistor-based polyamine sensor according to Item 15 or 16, wherein the organic compound is represented by the following formula (2): 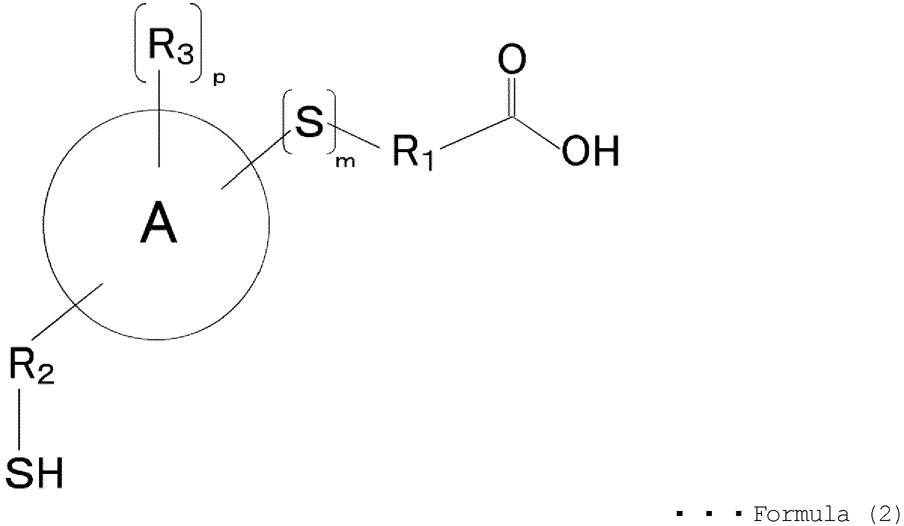 wherein
   a ring A represents a monocyclic heterocycle containing at least one member selected from the group consisting of a nitrogen atom, a sulfur atom, and an oxygen atom;
   R₁ represents an optionally substituted alkylene group having 1 to 10 carbon atoms;
   R₂ represents a single bond or an optionally substituted alkylene group having 1 to 10 carbon atoms;
   p is 0 or 1;
   when p is 1, R₃ represents a hydrogen atom or an alkyl group having 1 to 10 carbon atoms; and
   m is 0 or 1.
Item 18. The transistor-based polyamine sensor according to Item 17, wherein the ring A in the formula (2) is a 5-membered or 6-membered monocyclic heterocycle containing 1 or 2 nitrogen atoms.
Item 19. The transistor-based polyamine sensor according to Item 17 or 18, wherein the ring A in the formula (2) is a 5-membered monocyclic aromatic heterocycle containing 2 nitrogen atoms.
Item 20. The transistor-based polyamine sensor according to any one of Items 17 to 19, wherein the ring A in the formula (2) is thiadiazole.
Item 21. The transistor-based polyamine sensor according to any one of Items 17 to 20, wherein R₃ in the formula (2) is a hydrogen atom.
Item 22. The transistor-based polyamine sensor according to any one of Items 15 to 21, wherein the polyamine is at least one member selected from the group consisting of ethylenediamine, spermine, and spermidine.
Item 23. The transistor-based polyamine sensor according to any one of Items 15 to 21, wherein the polyamine is spermine and/or spermidine.
Item 24. The transistor-based polyamine sensor according to any one of Items 15 to 23, wherein the heavy metal ion is at least one member selected from the group consisting of a divalent iron ion, a divalent cobalt ion, a divalent zinc ion, a divalent cadmium ion, a divalent lead ion, a divalent copper ion, and a divalent nickel ion.
Item 25. The transistor-based polyamine sensor according to any one of Items 15 to 24, wherein the transistor is an organic thin-film transistor.
Item 26. A polyamine detection method comprising bringing the transistor-based polyamine sensor of any one of Items 15 to 25 into contact with a sample containing a polyamine.
Item 27. Use of an organic compound containing a carboxy group and a thiol group as a transistor-based polyamine sensor.

### Advantageous Effects of Invention

According to the present invention, a transistor-based polyamine sensor capable of detecting a polyamine easily and with high selectivity can be provided.

### Brief Description of Drawings

Fig. 1 is a schematic cross-sectional view of an example of the transistor-based polyamine sensor according to the present invention.
Fig. 2 is a graph showing the results of Fourier transform infrared (FT-IR) spectroscopy measurement (horizontal axis: wavenumber (cm⁻¹), vertical axis: absorbance).
Fig. 3 shows photographs showing the results of contact angle measurement.
Fig. 4 is a graph obtained by plotting the amount of change in the threshold voltage and the concentration of a divalent copper ion.
Fig. 5 is a graph showing the relationship between the gate voltage and the absolute value of the drain current associated with the addition of spermine in Example 1 (dashed line: before addition, solid line: after addition of spermine (150 µM)).
Fig. 6 is a graph showing the relationship between the amount of change in the threshold voltage and the concentration of spermine in Example 1.
Fig. 7 is a graph showing the relationship between the gate voltage and the absolute value of the drain current associated with the addition of spermidine in Example 2 (dashed line: before addition, solid line: after addition of spermidine (150 µM)).
Fig. 8 is a graph showing the relationship between the amount of change in the threshold voltage and the concentration of spermidine in Example 2.
Fig. 9 is a graph showing the relationship between the gate voltage and the absolute value of the drain current associated with the addition of ethylenediamine in Example 3 (dashed line: before addition, solid line: after addition of ethylenediamine (150 µM)).
Fig. 10 is a graph showing the relationship between the amount of change in the threshold voltage and the concentration of ethylenediamine in Example 3.
Fig. 11 is a graph showing the relationship between the gate voltage and the absolute value of the drain current associated with the addition of spermine in Example 4 (dashed line: before addition, solid line: after addition of spermine (150 µM)).
Fig. 12 is a graph showing the relationship between the amount of change in the threshold voltage and the concentration of spermine in Example 4.
Fig. 13 is a graph showing the relationship between the gate voltage and the absolute value of the drain current associated with the addition of spermine in Example 5 (dashed line: before addition, solid line: after addition of spermine (150 µM)).
Fig. 14 is a graph showing the relationship between the amount of change in the threshold voltage and the concentration of spermine in Example 5.
Fig. 15 is a graph showing the relationship between the gate voltage and the absolute value of the drain current associated with the addition of 1,6-diaminohexane in Example 6 (dashed line: before addition, solid line: after addition of 1,6-diaminohexane (150 µM)).
Fig. 16 is a graph showing the relationship between the amount of change in the threshold voltage and the concentration of 1,6-diaminohexane in Example 6.
Fig. 17 is a graph showing the relationship between the gate voltage and the absolute value of the drain current associated with the addition of cadaverine in Example 7 (dashed line: before addition, solid line: after addition of cadaverine (150 µM)).
Fig. 18 is a graph showing the relationship between the amount of change in the threshold voltage and the concentration of cadaverine in Example 7.
Fig. 19 is a graph showing the relationship between the gate voltage and the absolute value of the drain current associated with the addition of putrescine in Example 8 (dashed line: before addition, solid line: after addition of putrescine (150 µM)).
Fig. 20 is a graph showing the relationship between the amount of change in the threshold voltage and the concentration of putrescine in Example 8.
Fig. 21 is a graph showing the relationship between the gate voltage and the absolute value of the drain current associated with the addition of trimethylenediamine in Example 9 (dashed line: before addition, solid line: after addition of trimethylenediamine (150 µM)).
Fig. 22 is a graph showing the relationship between the amount of change in the threshold voltage and the concentration of trimethylenediamine in Example 9.
Fig. 23 is a graph showing the relationship between the gate voltage and the absolute value of the drain current associated with the addition of histamine in Example 10 (dashed line: before addition, solid line: after addition of histamine (150 µM)).
Fig. 24 is a graph showing the relationship between the amount of change in the threshold voltage and the concentration of histamine in Example 10.
Fig. 25 is a graph showing the relationship between the gate voltage and the absolute value of the drain current associated with the addition of tryptamine in Example 11 (dashed line: before addition, solid line: after addition of tryptamine (150 µM)).
Fig. 26 is a graph showing the relationship between the amount of change in the threshold voltage and the concentration of tryptamine in Example 11.
Fig. 27 is a graph showing the relationship between the gate voltage and the absolute value of the drain current associated with the addition of methylamine in Comparative Example 1 (dashed line: before addition, solid line: after addition of methylamine (150 µM)).
Fig. 28 is a graph showing the relationship between the amount of change in the threshold voltage and the concentration of methylamine in Comparative Example 1.
Fig. 29 is a graph showing the results of identifying nine polyamines (ethylenediamine, spermine, spermidine, 1,6-diaminohexane, cadaverine, putrescine, trimethylenediamine, histamine, and tryptamine) by LDA combined with a jackknife method in Example 12.
Fig. 30 is a graph showing the results of a quantitative detection test for spermine in mango juice in Example 13 (horizontal axis: the concentration of spermine in the liquid sample, vertical axis: the concentration of spermine predicted by SVM, ■: a calibration plot, ●: a predicted value plot of spermine at unknown concentration).

### Description of Embodiments

Preferred embodiments of the present invention are described below in detail. The structural features may be described below with reference to representative embodiments and specific examples. However, the present invention is not limited to such embodiments.

In the graduated numerical ranges described in the present specification, the upper or lower limit of the numerical range of one instance can be freely combined with the upper or lower limit of the numerical range of another instance. In the numerical ranges described herein, the upper or lower limit of the numerical ranges may be replaced with a value shown in the Examples or with a value that can be unambiguously derived from the Examples. In the present specification, numerical values connected by "to" mean a numerical range including the numerical values before and after "to" as the lower and upper limits.

In the present specification, the terms "comprising," "containing," and "including" include the concepts of comprising, containing, consisting essentially of, and consisting of.

In the present specification, "A and/or B" means "either A or B" or "both A and B." More specifically, "A and/or B" means "A," "B," or "A and B."

### 1. Transistor-based Polyamine Sensor

The transistor-based polyamine sensor according to the present invention comprises a detection site and is configured such that an organic compound containing a carboxy group and a thiol group is immobilized on all or part of the surface of the detection site via the thiol group of the organic compound; a heavy metal ion is bound to the carboxy group of the organic compound. The transistor-based polyamine sensor of the present invention detects a polyamine by measuring a change in threshold voltage caused by bonding of the heavy metal ion to the polyamine.

Based on these structural features, the transistor-based polyamine sensor of the present invention can detect a polyamine easily and with high sensitivity. Further, the transistor-based polyamine sensor of the present invention can be easily produced.

The transistor-based polyamine sensor of the present invention may be simply referred to below as "the present invention." In the present invention, an organic compound containing a carboxy group and a thiol group may be simply referred to as "the organic compound."

In the present invention, the polyamine to be detected is preferably at least one member selected from the group consisting of ethylenediamine, spermine, spermidine, 1,6-diaminohexane, cadaverine, putrescine, trimethylenediamine, histamine, and tryptamine; and is more preferably ethylenediamine, spermine, spermidine, 1,6-diaminohexane, cadaverine, putrescine, trimethylenediamine, histamine, and tryptamine.

The present invention preferably comprises a transistor and a detection site, wherein a gate electrode of the transistor is electrically connected to the detection site.

In the present invention, the detection site is preferably a detection electrode, and more preferably an extended-gate electrode.

In the present invention, the detection site preferably comprises a substrate and a noble metal thin film formed on the substrate. Examples of the noble metal include gold, silver, copper, ruthenium, rhodium, palladium, osmium, iridium, and platinum. Among these, gold, silver, palladium, and platinum are preferred, and gold is more preferred. The noble metal thin film is preferably formed on the substrate by a vacuum evaporation method. The thickness of the noble metal thin film is usually 70 nm to 100 nm. The material for the substrate at the detection site is not particularly limited, and a wide variety of materials commonly used in this technical field can be used. Examples of such materials include polyethylene terephthalate (PET), polyethylene naphthalate (PEN), polyethersulfone (PES), polyimide (PI), polyetheretherketone (PEEK), polycarbonate (PC), polyphenylene sulfide (PPS), polyetherimide (PEI), and the like.

In the present invention, all of the surface of the detection site means 1000 of the surface of the detection site. In the present invention, the part of the surface of the detection site usually refers to 1% or more and less than 1000 of the surface of the detection site, preferably 10% or more and 99.9% or less, more preferably 20% or more and 99.9% or less, and still more preferably 25% or more and 99.9% or less.

In the present invention, an organic compound containing a carboxy group and a thiol group is immobilized on all or part of the surface of the detection site via the thiol group of the organic compound. For example, subjecting the metal surface of the detection site to a self-assembled monolayer (SAM) treatment using an organic compound containing a carboxy group and a thiol group can form a state in which the organic compound is immobilized on all or part of the metal surface of the detection site via the thiol group of the organic compound. (That is, the hydrogen atom of the thiol group at the end of the organic compound is removed and a sulfur atom binds to all or part of the metal surface of the detection site to form a structure of "detection site metal surface-S-organic compound".)

The present invention is preferably configured to form a SAM having a structure of "detection site surface-S-organic compound" on all or part of the surface of the detection site. The present invention more preferably has a structural feature that a SAM having a structure of "gold thin film-S-organic compound" is formed on all or part of the surface of the gold thin film.

Since the organic compound containing a carboxy group and a thiol group is immobilized on all or part of the surface of the detection site via the thiol group of the organic compound, the organic compound is stable.

In the present invention, a heavy metal ion is bound to the carboxy group of the organic compound. The bond is preferably a coordinate bond. In the present invention, a heavy metal ion is preferably coordinated to the carboxy group of the organic compound. The heavy metal ion is preferably at least one member selected from the group consisting of a divalent iron ion, a divalent cobalt ion, a divalent zinc ion, a divalent cadmium ion, a divalent lead ion, a divalent copper ion, and a divalent nickel ion, and more preferably a divalent cobalt ion, a divalent lead ion, and a divalent copper ion, and particularly preferably a divalent copper ion.

In the present invention, a divalent copper ion is more preferably bound to the carboxy group of the organic compound. In the present invention, it is particularly preferred that a divalent copper ion is coordinated to the carboxy group of the organic compound, and the carboxy group and the divalent copper ion form a complex.

The present invention detects a polyamine by measuring a change in threshold voltage caused by bonding of a complex, in which a heavy metal ion is bound to a carboxy group of an organic compound, to the polyamine. Preferably, the present invention detects a polyamine by measuring a change in threshold voltage caused by bonding of a complex, in which a heavy metal ion is bound to a carboxy group of an organic compound, to the polyamine and extraction of the heavy metal ion by the polyamine.

More preferably, the present invention detects a polyamine by measuring a change in threshold voltage caused by bonding of a complex, which is formed by coordination bonding of a divalent copper ion to the carboxy group of an organic compound, to the polyamine. Still more preferably, the present invention detects a polyamine by measuring a change in threshold voltage caused by bonding of a complex, which is formed by bonding of a divalent copper ion to the carboxy group of an organic compound, to the polyamine and extraction of the divalent copper ion by the polyamine.

In the present invention, the organic compound preferably has a monocyclic heterocycle containing at least one member selected from the group consisting of a nitrogen atom, a sulfur atom, and an oxygen atom.

In the present invention, the organic compound can be classified into Embodiment 1 and Embodiment 2 below as preferred embodiments.

### Embodiment 1

In the present invention, the organic compound is preferably an organic compound represented by the following formula (1). 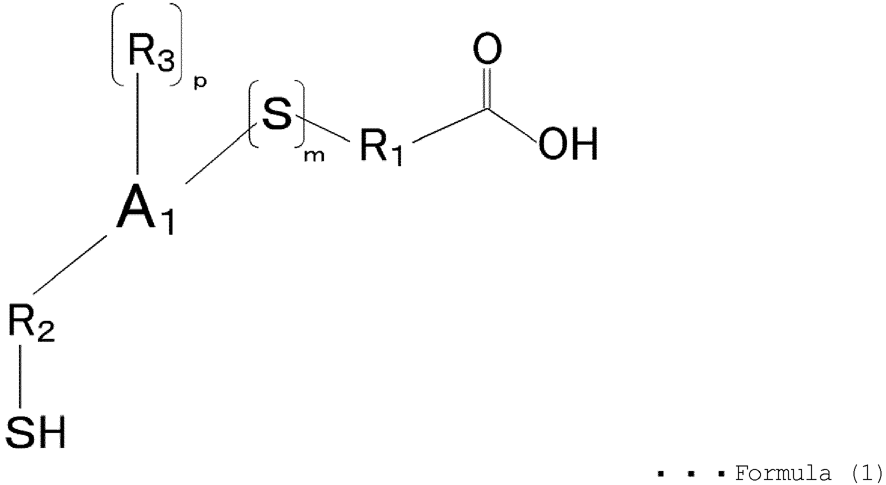 (In the formula (1), A₁ represents an aromatic hydrocarbon group, an aliphatic hydrocarbon group having 1 to 10 carbon atoms, or a monocyclic heterocyclic group containing at least one member selected from the group consisting of a nitrogen atom, a sulfur atom, and an oxygen atom;
R₁ represents an optionally substituted alkylene group having 1 to 10 carbon atoms;
R₂ represents a single bond or an optionally substituted alkylene group having 1 to 10 carbon atoms;
p is 0 or 1;
when p is 1, R₃ represents a hydrogen atom or an alkyl group having 1 to 10 carbon atoms; and
m is 0 or 1).

In the formula (1), the monocyclic heterocyclic group represented by A₁ is preferably a 5-membered or 6-membered monocyclic heterocyclic group containing 1 or 2 nitrogen atoms, and more preferably a 5-membered monocyclic aromatic heterocyclic ring containing 2 nitrogen atoms.

In the formula (1), the monocyclic heterocyclic group represented by A₁ is preferably pyridine, pyridazine, pyrimidine, pyrazine, triazine, pyrrole, pyrazole, imidazole, triazole, tetrazole, furan, thiophene, oxazole, oxadiazole, thiazole, or thiadiazole, and more preferably thiadiazole. That is, the organic compound represented by formula (1) is more preferably a thiadiazole compound containing a carboxy group and a thiol group.

In the formula (1), the aromatic hydrocarbon group represented by A₁ is preferably a phenyl group, a naphthyl group, or an anthracenyl group, more preferably a phenyl group or a naphthyl group, and still more preferably a phenyl group.

In the formula (1), the aliphatic hydrocarbon group having 1 to 10 carbon atoms (1, 2, 3, 4, 5, 6, 7, 8, 9, or 10 carbon atoms) represented by A₁ is preferably an alkyl group having 1 to 10 carbon atoms, an alkenyl group having 2 to 10 carbon atoms, or an alkynyl group having 2 to 10 carbon atoms, more preferably an alkyl group having 1 to 10 carbon atoms, and still more preferably an alkenyl group having 1 to 3 carbon atoms (1, 2, or 3 carbon atoms).

Examples of the alkyl group having 1 to 10 carbon atoms include methyl, ethyl, n-propyl, isopropyl, n-butyl, isobutyl, sec-butyl, tert-butyl, n-hexyl, n-heptyl, n-octyl, n-nonyl, n-decyl, and the like.

Examples of the alkenyl group having 2 to 10 carbon atoms include vinyl, 1-propenyl, 2-propenyl, isopropenyl, 1-butenyl, 2-butenyl, 1,3-butadienyl, 1-pentenyl, 2-pentenyl, 1-hexenyl, 2-ethyl-2-butenyl, 2-octenyl, and the like.

Examples of the alkynyl group having 2 to 10 carbon atoms include ethynyl, 1-propynyl, 1-butynyl, 1-pentynyl, 3-pentynyl, 1-hexynyl, 2-ethyl-2-butynyl, 2-octynyl, and the like.

In the formula (1), R₁ is preferably an optionally substituted alkylene group having 1 to 6 carbon atoms (1, 2, 3, 4, 5, or 6 carbon atoms), more preferably an optionally substituted alkylene group having 1 to 3 carbon atoms (1, 2, or 3 carbon atoms), still more preferably an alkylene group having 1 to 3 carbon atoms (1, 2, or 3 carbon atoms), and particularly preferably an alkylene group having 1 carbon atom (-CH₂-). The "substituent" is not particularly limited as long as it is not a hydrogen atom. Examples include halogen atoms (fluorine, chlorine, bromine, and iodine atoms), a hydroxy group, an amino group, a phenyl group, and the like. When such substituents are present, the number of substituents is usually 1 to 4, preferably 1 or 2, and more preferably 1.

In the formula (1), R₂ is preferably a single bond or an optionally substituted alkylene group having 1 to 6 carbon atoms (1, 2, 3, 4, 5, or 6 carbon atoms), more preferably a single bond or an optionally substituted alkylene group having 1 to 3 carbon atoms (1, 2, or 3 carbon atoms), and still more preferably a single bond (that is, a thiol group is directly bound to A₁). The "substituent" is not particularly limited as long as it is not a hydrogen atom. Examples include halogen atoms (fluorine, chlorine, bromine, and iodine atoms), a hydroxy group, an amino group, a phenyl group, and the like. When such substituents are present, the number of substituents is usually 1 to 4, preferably 1 or 2, and more preferably 1.

In the formula (1), when p is 1, R₃ is preferably a hydrogen atom or an alkyl group having 1 to 6 carbon atoms (1, 2, 3, 4, 5, or 6 carbon atoms), more preferably a hydrogen atom or an alkyl group having 1 to 3 carbon atoms (1, 2, or 3 carbon atoms), and still more preferably a hydrogen atom. The alkyl group may further have a substituent. The "substituent" is not particularly limited as long as it is not a hydrogen atom. Examples include halogen atoms (fluorine, chlorine, bromine, and iodine atoms), a hydroxy group, an amino group, a phenyl group, and the like. When such substituents are present, the number of substituents is usually 1 to 4, preferably 1 or 2, and more preferably 1. In the formula (1), p is particularly preferably 0.

In the formula (1), m is preferably 1.

In Embodiment 1, the organic compound containing a carboxy group and a thiol group is preferably 2-thioacetic acid-5-mercapto-1,3,4-thiadiazole (TMT), 4-mercaptobenzoic acid, and 5-carboxy-1-pentanethiol, more preferably TMT and 4-mercaptobenzoic acid, and particularly preferably TMT.

### Embodiment 2

In the present invention, the organic compound is preferably an organic compound represented by the following formula (2). 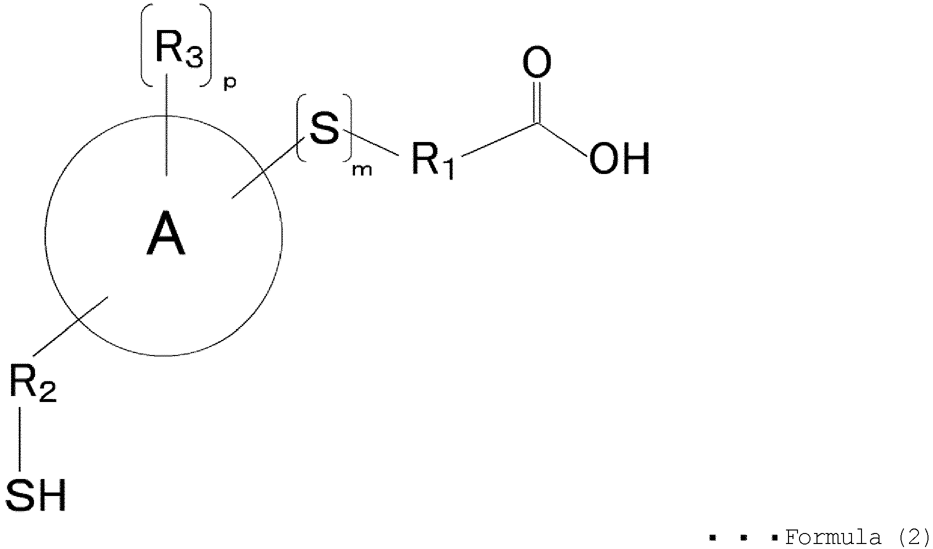 (In the formula (2), a ring A represents a monocyclic heterocycle containing at least one member selected from the group consisting of a nitrogen atom, a sulfur atom, and an oxygen atom,
R₁ represents an optionally substituted alkylene group having 1 to 10 carbon atoms,
R₂ represents a single bond or an optionally substituted alkylene group having 1 to 10 carbon atoms,
p is 0 or 1,
when p is 1, R₃ represents a hydrogen atom or an alkyl group having 1 to 10 carbon atoms, and
m is 0 or 1.

In the formula (2), the ring A is preferably a 5-membered or 6-membered monocyclic heterocycle containing 1 or 2 nitrogen atoms, and more preferably a 5-membered monocyclic aromatic heterocycle containing 2 nitrogen atoms. In the formula (2), the ring A is preferably pyridine, pyridazine, pyrimidine, pyrazine, triazine, pyrrole, pyrazole, imidazole, triazole, tetrazole, furan, thiophene, oxazole, oxadiazole, thiazole, or thiadiazole, and more preferably thiadiazole. That is, the organic compound represented by formula (2) is more preferably a thiadiazole compound containing a carboxy group and a thiol group.

In the formula (2), R₁ is preferably an optionally substituted alkylene group having 1 to 6 carbon atoms (1, 2, 3, 4, 5, or 6 carbon atoms), and more preferably an optionally substituted alkylene group having 1 to 3 carbon atoms (1, 2, or 3 carbon atoms), and still more preferably an alkylene group having 1 carbon atom (-CH₂-) . The "substituent" is not particularly limited as long as it is not a hydrogen atom. Examples include halogen atoms (fluorine, chlorine, bromine, and iodine atoms), a hydroxy group, an amino group, a phenyl group, and the like. When such substituents are present, the number of substituents is usually 1 to 4, preferably 1 or 2, and more preferably 1.

In the formula (2), R₂ is preferably a single bond or an optionally substituted alkylene group having 1 to 6 carbon atoms (1, 2, 3, 4, 5, or 6 carbon atoms), more preferably a single bond or an alkylene group having 1 to 3 carbon atoms (1, 2, or 3 carbon atoms), and still more preferably a single bond (that is, the thiol group is directly bound to the ring A). The "substituent" is not particularly limited as long as it is not a hydrogen atom. Examples include halogen atoms (fluorine, chlorine, bromine, and iodine atoms), a hydroxy group, an amino group, a phenyl group, and the like. When such substituents are present, the number of substituents is usually 1 to 4, preferably 1 or 2, and more preferably 1.

In the formula (2), when p is 1, R₃ is preferably a hydrogen atom or an alkyl group having 1 to 6 carbon atoms (1, 2, 3, 4, 5, or 6 carbon atoms), more preferably a hydrogen atom or an alkyl group having 1 to 3 carbon atoms (1, 2, or 3 carbon atoms), and still more preferably a hydrogen atom. The alkyl group may further have a substituent. The "substituent" is not particularly limited as long as it is not a hydrogen atom. Examples include halogen atoms (fluorine, chlorine, bromine, and iodine atoms), a hydroxy group, an amino group, a phenyl group, and the like. When such substituents are present, the number of substituents is usually 1 to 4, preferably 1 or 2, and more preferably 1. In the formula (2), p is particularly preferably 0.

In the formula (2), m is preferably 1.

In Embodiment 2, it is particularly preferred that the organic compound containing a carboxy group and a thiol group is 2-thioacetic acid-5-mercapto-1,3,4-thiadiazole (TMT).

Preferably, the present invention further comprises a reference electrode. When the present invention comprises a reference electrode, a relative change amount (difference) of the target substance can be understood, for example, from a plurality of measured values or measured values over time. When the present invention comprises a reference electrode, the reference electrode is preferably a silver/silver chloride electrode (Ag/AgCl electrode), a gold electrode treated with a self-assembled monolayer (SAM treatment), or a metal coated with a polymer. When the present invention comprises such a reference electrode, absolute evaluation of the target substance can be made.

The transistor site of the transistor-based polyamine sensor of the present invention (hereinafter sometimes simply referred to as "transistor site of the present invention") can be constituted by a known transistor structure. The transistor site may be an inorganic transistor or an organic transistor. Among these, a thin-film transistor (TFT) is preferred because it is small and can be easily used. In this case, a flexible polyamine sensor can be formed by using, as a material for the substrate, an inorganic material, such as glass, ceramics, or metals, or an organic material, such as resin or paper.

Examples of methods for producing a TFT include dry processes such as evaporation method and sputtering; application by spin coating, bar coating, spray coating, etc.; and printing by various printing machines, such as screen printing, gravure offset printing, letterpress reverse printing, and inkjet printing. Among these, printing allows for more efficient low-cost production.

The transistor site of the present invention is more preferably an organic thin film transistor (organic TFT, OTFT). When the transistor site of the present invention is an organic TFT, the substrate may be formed by using, for example, a resin such as polyethylene naphthalate, polyethylene terephthalate, polyethylene, polyimide, or polyparaxylylene (parylene (registered trademark)), or paper.

When the transistor site of the present invention is an organic TFT, examples of constituent materials for P-type organic semiconductors include pentacene, dinaphthothienothiophene, benzothienobenzothiophene (Cn-BTBT), TIPS pentacene, TES-ADT, rubrene, P3HT, PBTTT, and the like. Examples of constituent materials for N-type organic semiconductors include fullerene and the like.

Examples of gate electrode materials in the transistor site of the present invention include aluminum, silver, gold, copper, titanium, ITO, PEDOT:PSS, and the like.

Examples of the source electrode material and the drain electrode material in the transistor site of the present invention include conductive polymers, such as gold, silver, copper, platinum, aluminum, and PEDOT:PSS.

Examples of constituent materials for the gate insulating film in the transistor site of the present invention include silica, alumina, self-assembled monolayers (SAM), polystyrene, polyvinyl phenol, polyvinyl alcohol, polymethyl methacrylate, polydimethylsiloxane, polysilsesquioxane, ionic liquids, polytetrafluoroethylene (Teflon (registered trademark) AF, Cytop (registered trademark)), and the like.

Examples of the constituent materials for a sealing film (protective film) in the transistor site of the present invention include polytetrafluoroethylene (Teflon (registered trademark) AF, Cytop (registered trademark)), polyparaxylene (Parylene (registered trademark)), and the like.

In the present invention, the transistor site and the detection site are separately produced and configured to be connected to each other when used, whereby only the detection site that is in direct contact with samples can be easily replaced in accordance with its service life and attached. This enables the transistor site to make measurements in a stable condition. Further, since it is unnecessary to replace the entire transistor-based polyamine sensor and the detection site can be repeatedly used by cleaning, the transistor-based polyamine sensor also has the advantage of being economical.

### 2. Polyamine Detection Method

The polyamine detection method of the present invention comprises the step of A) bringing a transistor-based polyamine sensor into contact with a sample containing a polyamine. The details of the transistor-based polyamine sensor are as described above in "1. Transistor-based Polyamine Sensor," unless otherwise specified. Examples of the polyamine to be detected include at least one member selected from the group consisting of ethylenediamine, spermine, spermidine, 1,6-diaminohexane, cadaverine, putrescine, trimethylenediamine, histamine, and tryptamine, and more preferably at least one member selected from the group consisting of ethylenediamine, spermine, spermidine, 1,6-diaminohexane, cadaverine, putrescine, trimethylenediamine, histamine, and tryptamine.

Data on transmission characteristics of the transistor-based polyamine sensor of the present invention when a polyamine is added (i.e., data on transmission characteristics of the transistor-based polyamine sensor of the present invention before and after the addition of polyamine) can be obtained by using the method for detecting a polyamine according to the present invention. The transistor site of the transistor-based polyamine sensor is preferably an organic thin-film transistor.

The contacting means in step A) above include, for example, immersion of all or part of the detection site of the transistor-based polyamine sensor into a solution containing polyamine. Immersion of all of the detection site means that the detection site of the transistor-based polyamine sensor of the present invention is completely immersed into a solution containing a polyamine. Immersion of part of the detection site means that the detection site of the transistor-based polyamine sensor of the invention is partially immersed into a solution containing a polyamine.

The polyamine detection method of the present invention preferably comprises, after step A), step B) of measuring a change in threshold voltage caused by bonding of a polyamine contained in a sample to the heavy metal ion described above to detect the polyamine.

### 3. Use

In the present invention, an organic compound containing a carboxy group and a thiol group can be used in the transistor-based polyamine sensor. The details of the organic compound containing a carboxy group and a thiol group and the transistor-based polyamine sensor in the "3. Use" section are the same as described above in "1. Transistor-based Polyamine Sensor," unless otherwise specified.

In the present invention, a thiadiazole compound containing a carboxy group and a thiol group is preferably used in the transistor-based polyamine sensor.

In the present invention, 2-thioacetic acid-5-mercapto-1,3,4-thiadiazole (TMT), 4-mercaptobenzoic acid, and 5-carboxy-1-pentanethiol are more preferably used in the transistor-based polyamine sensor. In the present invention, TMT and 4-mercaptobenzoic acid are still more preferably used in the transistor-based polyamine sensor. In the present invention, TMT is particularly preferably used in the transistor-based polyamine sensor.

### 4. Method for Identifying Polyamine

The method for identifying a polyamine according to the present invention comprises the step of (1) creating a model that has undergone pattern learning by linear discriminant analysis (LDA), based on data on a change in transmission characteristics of the transistor-based polyamine sensor, the data being obtained by using the polyamine detection method described above; and identifying the polyamine by pattern recognition. The details of the transistor-based polyamine sensor are the same as described above in "1. Transistor-based Polyamine Sensors," unless otherwise specified. The details of the polyamine detection method are the same as described above in "2. Polyamine Detection Method," unless otherwise specified.

In step (1), a model is usually a canonical score plot.

In step (1), LDA usually carries out cross-validation and data analysis simultaneously. Examples of the cross-validation include the jackknife method (leave-one-out) and the like.

In step (1), it is preferable to identify two or more polyamines (preferably ethylenediamine, spermine, spermidine, 1,6-diaminohexane, cadaverine, putrescine, trimethylenediamine, histamine, and tryptamine) by pattern recognition.

In the present invention, the method preferably comprises, after step (1), step (2) of measuring the concentration of the polyamine identified in step (1) by using a support vector machine (SVM), which is a type of machine learning model (hereafter referred to as "step (2)"). In the present invention, a combination of steps (1) and (2) may be also referred to as the method for measuring the polyamine concentration.

Step (2) is preferably a step in which an SVM is used to measure the concentration of one or more specific polyamines to be detected (preferably spermine and/or spermidine, more preferably spermine) among two or more polyamines identified by using the SVM in step (1) (preferably ethylenediamine, spermine, spermidine, 1,6-diaminohexane, cadaverine, putrescine, trimethylenediamine, histamine, and tryptamine).

In step (2), calibration curves prepared from calibration plots can be usually used to measure the concentration.

### 5. Method for Measuring the Polyamine Concentration

The method for measuring the polyamine concentration according to the present invention comprises the step of measuring the concentration of the polyamine to be measured in the presence of an impurity other than the polyamine to be measured, by using a support vector machine, which is a type of machine learning model, based on the data on a change in transmission characteristics of the transistor-based polyamine sensor, the data being obtained using the polyamine detection method described above. The details of the transistor-based polyamine sensor are the same as described above in "1. Transistor-based Polyamine Sensor," unless otherwise specified. The details of the polyamine detection method are the same as described above in "2. Polyamine Detection Method," unless otherwise specified. In the above step, calibration curves prepared from calibration plots can be usually used to measure the concentration.

The polyamine to be measured is preferably spermine and/or spermidine, and more preferably spermine.

The impurity preferably refers to polyamines other than the polyamine to be measured. In this case, polyamines as the impurity are a generic term for compounds that contain two or more amino groups and that are present in *E. coli* or animal cells in the order of several mM to several tens of mM, as described above in the Background Art section. Examples of polyamines as the impurity include ethylenediamine, 1,6-diaminohexane, cadaverine, putrescine, trimethylenediamine, histamine, tryptamine, propylenediamine, phenyldiamine, naphthalenediamine, xylenediamine, isophorone diamine, cardopentamine, cardine, theremin, theremospermine, theremospermine, canabalmin, and cardohexamine.

For example, when spermine is the polyamine to be measured, polyamines as the impurity are preferably spermidine and polyamines other than spermine. When spermine is the polyamine to be measured, polyamines as the impurity are more preferably (i) spermidine and at least one member selected from the group consisting of (ii) ethylenediamine, 1,6-diaminohexane, cadaverine, putrescine, trimethylenediamine, histamine, tryptamine, propylenediamine, phenyldiamine, naphthalenediamine, xylenediamine, isophoronediamine, cardopentamine, cardine, theremin, theremospermine, canavalamine, and cardohexamine.

When spermidine is the polyamine to be measured, polyamines as the impurity are preferably spermine and polyamines other than spermidine. When spermidine is the polyamine to be measured, polyamines as the impurity are more preferably (i) spermine and at least one member selected from the group consisting of (ii) ethylenediamine, 1,6-diaminohexane, cadaverine, putrescine, trimethylenediamine, histamine, tryptamine, propylenediamine, propylenediamine, phenyldiamine, naphthalenediamine, xylenediamine, isophoronediamine, cardopentamine, cardine, theremin, theremospermine, canavalamine, and cardohexamine.

### Examples

The present invention is described below in detail with reference to Production Examples and Examples. However, the present invention is not limited to the Production Examples and Examples.

### Production Example 1: Preparation of transistor-based polyamine sensor with immobilized TMT

A gate electrode 12 (aluminum (Al), thickness: 30 nm) was formed on a glass substrate 11, and on its surface, an aluminum oxide (AlOₓ) film was formed by reactive ion-etching (RIE) treatment. The resulting substrate was immersed in a tetradecylphosphonic acid solution to form a gate insulating film 13. Next, source and drain electrodes 14, 15 (gold (Au), thickness: 30 nm) were formed by patterning. Subsequently, a liquid-repellent bank 16 (Teflon (registered trademark) AF1600) was formed with a dispensing device, and a polymer semiconductor layer 17 (pBTTT-C₁₆) was formed by using a drop-cast method. A sealing film 18 (Cytop (registered trademark) CTL-809M) was formed on the substrate by a spin-coating method, thus preparing a transistor site 1.

Next, a gold thin film was formed on the surface of a PEN film substrate 21 (referred to below as "the substrate 21") by a vacuum evaporation method. A methanol solution was obtained by dissolving 10 mM 2-thioacetic acid-5-mercapto-1,3,4-thiadiazole (TMT) in methanol. By immersing the substrate 21 in the methanol solution at 25°C for 1 hour, the gold thin film on the surface of the substrate 21 (referred to below as "the gold thin film on the substrate 21") was subjected to self-assembled monolayer (SAM) treatment using TMT.

### Fourier transform infrared (FT-IR) spectroscopy measurement and contact angle measurement

Fourier transform infrared (FT-IR) spectroscopy measurement and contact angle measurement were performed to confirm whether TMT was immobilized on the SAM-treated gold thin film on the substrate 21 via the thiol group.

The FT-IR spectroscopy measurement was performed with an FT-IR spectrometer (product name: Nicolet iS-5, produced by Thermo Fisher Scientific K.K.) under the following measurement conditions.

### Measurement conditions

Measurement method: ATR method
Measuring temperature: 25°C
Prism: Germanium
Detector: DTGS

For the contact angle measurement, the contact angle of water was measured with a contact angle meter (product name: CA-X, produced by Kyowa Interface Science Co., Ltd.) under the following measurement conditions.

### Measurement conditions

Solvent used: pure water
Measuring temperature: 25°C

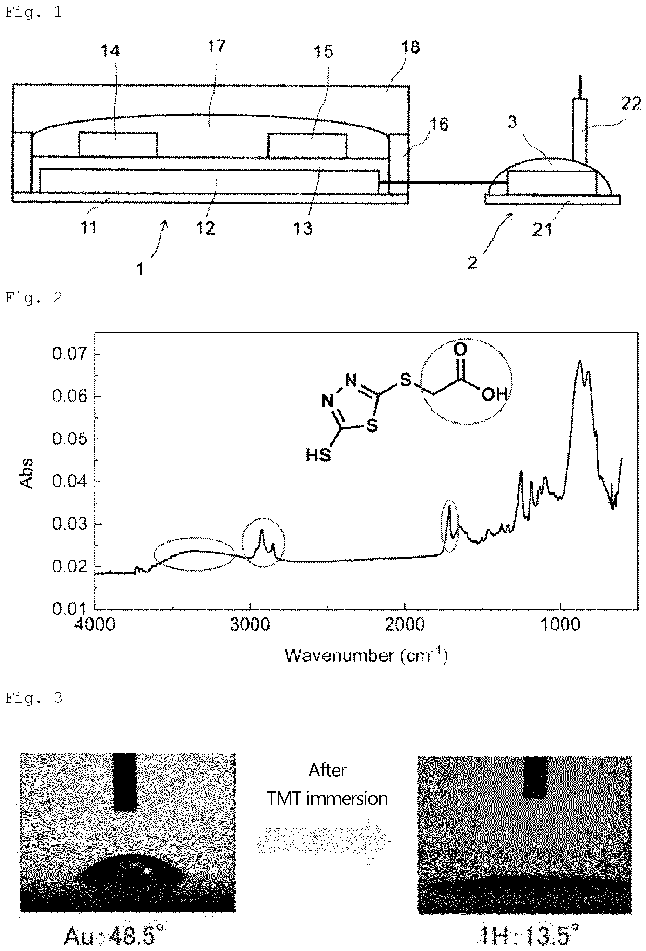

Fig. 2 shows the FT-IR spectral results and the structural formula of TMT. A peak derived from the C=O of the carboxy group of TMT was observed at around 1711 cm⁻¹, peaks derived from the C-H of TMT were observed at around 2852 cm⁻¹ and 2922 cm⁻¹, and a peak derived from the O-H of the carboxy group of TMT was observed at around 3000 to 3300 cm⁻¹.

Fig. 3 shows photographs of a water droplet in contact with the gold thin film on the substrate 21 before TMT immersion (photo on the left in Fig. 3) and after TMT immersion (photo on the right in Fig. 3). The angle between the gold thin film surface and a water droplet is called the "contact angle." The contact angle before TMT immersion was 48.5°, whereas the contact angle after TMT immersion was 13.5°.

The results of the FT-IR spectroscopy measurement and contact angle measurement confirm that TMT was immobilized via the thiol group on the SAM-treated gold thin film on the substrate 21.

### Copper Ion Detection Test

After the FT-IR spectroscopy measurement and contact angle measurement, a transistor-based sensor was prepared by connecting the SAM-treated substrate 21 and the gate electrode of the transistor site 1. Then, a copper ion detection test was performed using the transistor-based sensor. A liquid sample for use was obtained by adding copper perchlorate (0 µM to 1000 µM) to a 100 mM HEPES buffer solution at a pH of 7.4.

A silver/silver chloride electrode (Ag/AgCl electrode) was used as a reference electrode 22.

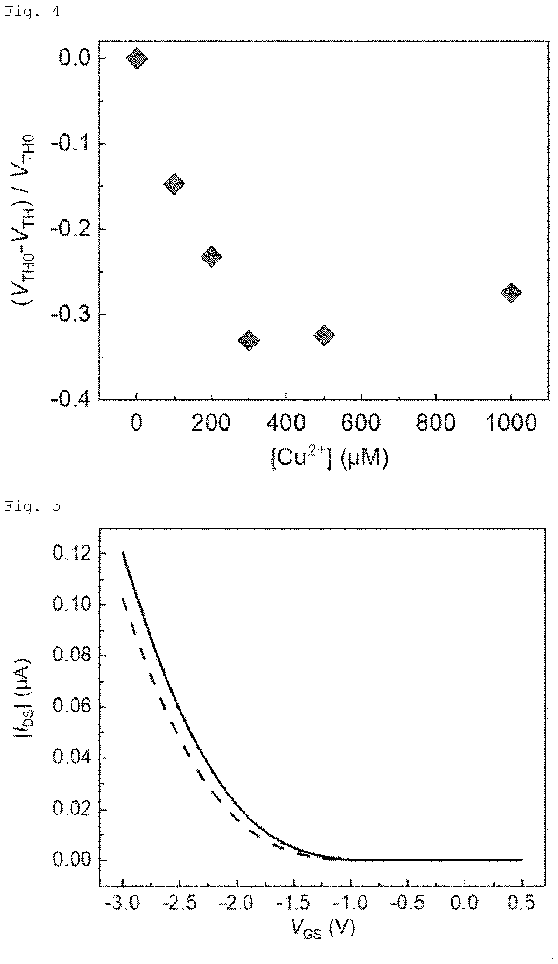

Fig. 4 is a graph obtained by plotting the amount of change in the threshold voltage (vertical axis) and the concentration of a divalent copper ion (horizontal axis).

The graph shown in Fig. 4 confirmed a change in the threshold voltage associated with the addition of a divalent copper ion, indicating that detection of a divalent copper ion was possible. Specifically, it was confirmed that the divalent-copper-ion coordination caused the detection electrode surface to have a positive electric charge, which in turn caused the threshold voltage to change in a negative direction. These results of the copper ion detection test confirmed that a divalent copper ion coordinated to the carboxy group of the TMT immobilized via the thiol group on the SAM-treated gold thin film on the substrate 21 to thus form a complex of the carboxy group and the divalent copper ion.

After the copper ion detection test, the substrate 21 connected to the gate electrode of the transistor site 1 was immersed in a 100 mM HEPES buffer solution (containing 1 mM copper perchlorate) at a pH of 7.4 for 1 hour at 25°C, thus preparing a transistor-based polyamine sensor with immobilized TMT for use in Examples 1 and 2 below.

### Production Example 2: Preparation of transistor-based polyamine sensor with immobilized 4-mercaptobenzoic acid

A transistor-based polyamine sensor was prepared in the same manner as in Production Example 1, except that the self-assembled monolayer (SAM) treatment was performed using 4-mercaptobenzoic acid.

Further, in the same manner as in Production Example 1, FT-IR spectroscopy measurement and contact angle measurement were performed to confirm whether 4-mercaptobenzoic acid was immobilized via the thiol group on the SAM-treated gold thin film on the substrate 21.

Furthermore, a copper ion detection test was performed in the same manner as in Production Example 1 to confirm that a divalent copper ion coordinated to the carboxy group of the 4-mercaptobenzoic acid immobilized via the thiol group on the SAM-treated gold thin film on the substrate 21 to thus form a complex of the carboxy group and the divalent copper ion.

After the copper ion detection test, the substrate 21 connected to the gate electrode of the transistor site 1 was immersed in a 100 mM HEPES buffer solution (containing 1 mM copper perchlorate) at a pH of 7.4 for 1 hour at 25°C, thus preparing a transistor-based polyamine sensor with immobilized 4-mercaptobenzoic acid for use in Example 3 below.

### Production Example 3: Preparation of transistor-based polyamine sensor with immobilized 5-carboxy-1-pentanethiol

A transistor-based polyamine sensor was produced in the same manner as in Production Example 1, except that the self-assembled monolayer (SAM) treatment was performed using 5-carboxy-1-pentanethiol.

Further, in the same manner as in Production Example 1, FT-IR spectroscopy measurement and contact angle measurement were performed to confirm whether 5-carboxy-1-pentanethiol was immobilized via the thiol group on the SAM-treated gold thin film on the substrate 21.

Furthermore, a copper ion detection test was performed in the same manner as in Production Example 1 to confirm that a divalent copper ion coordinated to the carboxy group of the 5-carboxy-1-pentanethiol immobilized via the thiol group on the SAM-treated gold thin film on the substrate 21 to thus form a complex of the carboxy group and the divalent copper ion.

After the copper ion detection test, the substrate 21 connected to the gate electrode of the transistor site 1 was immersed in a 100 mM HEPES buffer solution (containing 1 mM copper perchlorate) at a pH of 7.4 for 1 hour at 25°C, thus preparing a transistor-based polyamine sensor with immobilized 5-carboxy-1-pentanethiol for use in Example 4 below.

### Example 1: Spermine detection test using transistor-based polyamine sensor with TMT

A spermine detection test was performed using the transistor-based polyamine sensor prepared in Production Example 1. The detection test was performed by immersing the detection electrode of the transistor-based polyamine sensor prepared in Production Example 1 in a liquid sample, which is a measurement solution, allowing it to stand at 25°C for 10 minutes, and then measuring the transmission characteristics of the organic thin film transistor electrically connected to the detection electrode with a semiconductor parameter analyzer. The measurement conditions were such that the gate voltage (V_{GS}) = -3.0 to 0.5 V, and the drain voltage (V_{DS}) = -1.0 V. The liquid sample used here was obtained by adding spermine (0 to 150 µM)to a 50 mM CHES buffer solution (containing 10 mM NaCl) at a pH of 8.5.

Fig. 5 is a graph showing the relationship between the gate voltage (V_{GS}) and the absolute value of drain current (I_{DS}) associated with the addition of spermine (dashed line: before addition, solid line: after addition of spermine (150 µM)). The graph shown in Fig. 5 confirmed a change in the transmission characteristics of the organic thin film transistor associated with the addition of spermine, indicating that spermine detection was possible. The positive shift of the gate voltage is assumed to be a response caused by spermine extracting the copper ion coordinately bonded to 2-thioacetic acid-5-mercapto-1,3,4-thiadiazole (TMT) in the self-assembled monolayer, associated with the addition of spermine. The response reached saturation in about 5 minutes, and the response was confirmed to be stable thereafter.

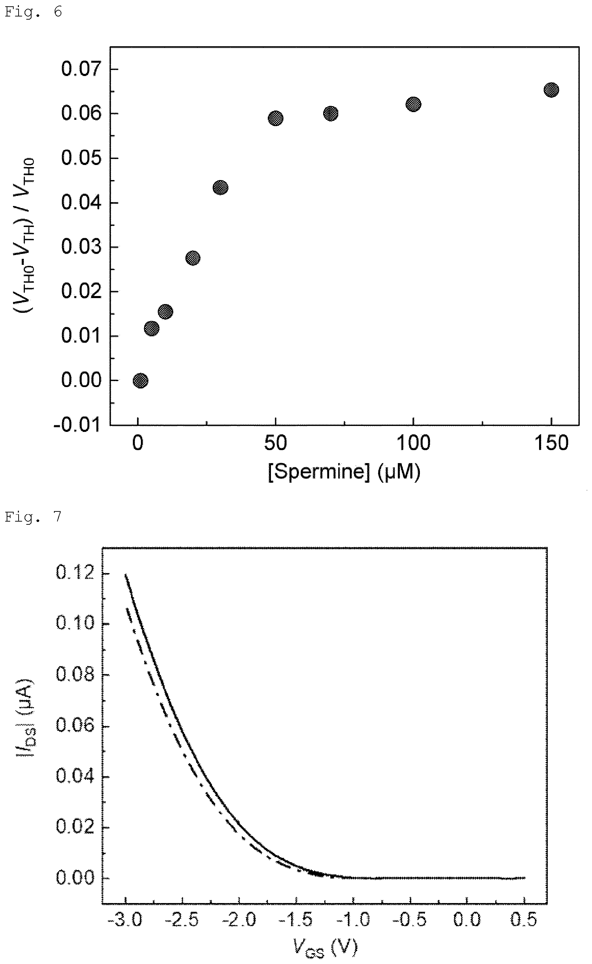

Fig. 6 is a graph showing the relationship between the amount of change in the threshold voltage and the spermine concentration. The graph shown in Fig. 6 confirmed a change in the threshold voltage associated with a change in the spermine concentration, indicating that detection of a change in the spermine concentration was possible. The response reached saturation at 50 µM, indicating that spermine detection was possible at a low concentration.

### Example 2: Spermidine detection test using transistor-based polyamine sensor with TMT

A spermidine detection test was performed using the transistor-based polyamine sensor prepared in Production Example 1. The detection test was performed by immersing the detection electrode of the transistor-based polyamine sensor prepared in Production Example 1 in a liquid sample, which is a measurement solution, allowing it to stand at 25°C for 10 minutes, and then measuring the transmission characteristics of the organic thin film transistor electrically connected to the detection electrode with a semiconductor parameter analyzer. The measurement conditions were such that the gate voltage (V_{GS}) = -3.0 to 0.5 V, and the drain voltage (V_{DS}) = -1.0 V. The liquid sample used here was obtained by adding spermidine (0 to 150 µM) to a 50 mM CHES buffer solution (containing 10 mM NaCl) at a pH of 8.5.

Fig. 7 is a graph showing the relationship between the gate voltage (V_{GS}) and the absolute value of drain current (I_{DS}) associated with the addition of spermidine (dashed line: before addition, solid line: after addition of spermidine (150 µM)). The graph shown in Fig. 7 confirmed a change in the transmission characteristics of the organic thin film transistor associated with the addition of spermidine, indicating that spermidine detection was possible. The positive shift of the gate voltage is assumed to be a response caused by spermidine extracting the copper ion coordinately bonded to TMT in the self-assembled monolayer, associated with the addition of spermidine. The response reached saturation in about 5 minutes, and the response was confirmed to be stable thereafter.

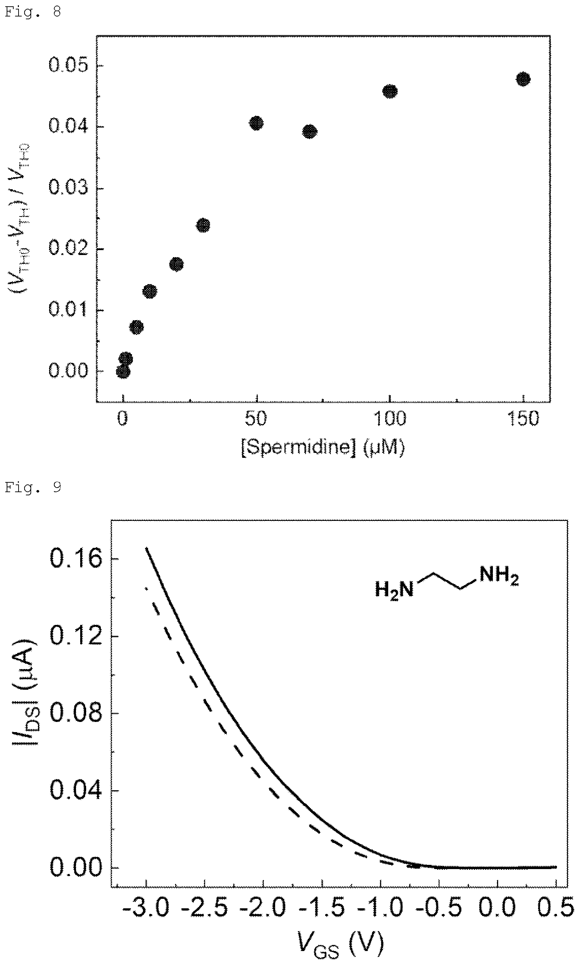

Fig. 8 is a graph showing the relationship between the amount of change in the threshold voltage and the spermidine concentration. The graph shown in Fig. 8 confirmed a change in the threshold voltage associated with a change in the spermidine concentration, indicating that detection of a change in the spermidine concentration was possible. The response reached saturation at 50 µM, indicating that spermidine detection was possible at a low concentration.

### Example 3: Ethylenediamine detection test using transistor-based polyamine sensor with TMT

An ethylenediamine detection test was performed using the transistor-based polyamine sensor prepared in Production Example 1. The detection test was performed by immersing the detection electrode of the transistor-based polyamine sensor prepared in Production Example 1 in a liquid sample, which is a measurement solution, allowing it to stand at 25°C for 10 minutes, and then measuring the transmission characteristics of the organic thin film transistor electrically connected to the detection electrode with a semiconductor parameter analyzer. The measurement conditions were such that the gate voltage (V_{GS}) = - 3.0 to 0.5 V, and the drain voltage (V_{DS}) = -1.0 V. The liquid sample used here was obtained by adding ethylenediamine (0 to 150 µM)to a 50 mM CHES buffer solution (containing 10 mM NaCl) at a pH of 8.5.

Fig. 9 is a graph showing the relationship between the gate voltage (V_{GS}) and the absolute value of drain current (I_{DS}) associated with the addition of ethylenediamine (dashed line: before addition, solid line: after addition of ethylenediamine (150 µM)). The graph shown in Fig. 9 confirmed a change in the transmission characteristics of the organic thin film transistor associated with the addition of ethylenediamine, indicating that ethylenediamine detection was possible. The positive shift of the gate voltage is assumed to be a response caused by ethylenediamine extracting the copper ion coordinately bonded to 2-thioacetic acid-5-mercapto-1,3,4-thiadiazole (TMT) in the self-assembled monolayer, associated with the addition of ethylenediamine. The response reached saturation in about 5 minutes, and the response was confirmed to be stable thereafter.

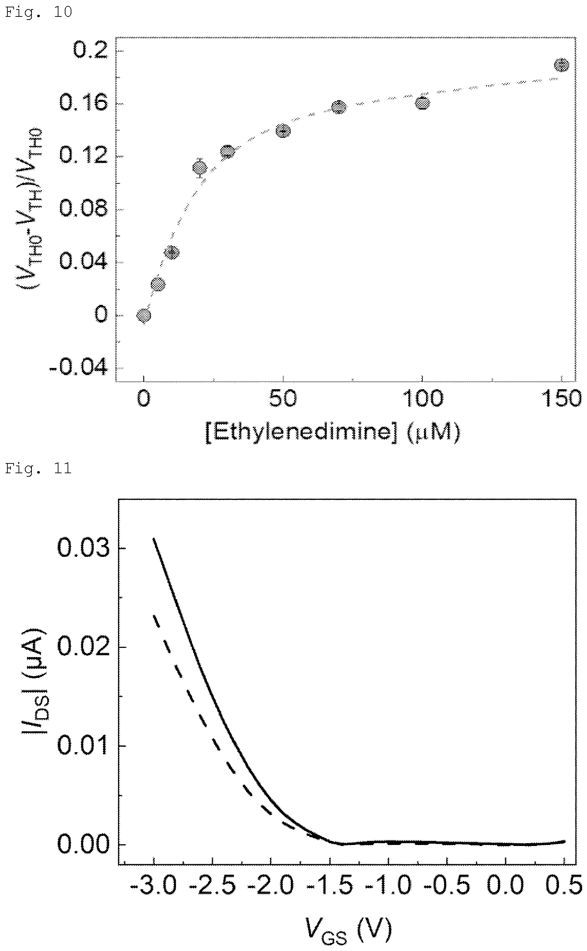

Fig. 10 is a graph showing the relationship between the amount of change in the threshold voltage and the ethylenediamine concentration. The graph shown in Fig. 10 confirmed a change in the threshold voltage associated with a change in the ethylenediamine concentration, indicating that detection of a change in the ethylenediamine concentration was possible. The response reached saturation at 50 µM, indicating that ethylenediamine detection was possible at a low concentration.

### Example 4: Spermine detection test using transistor-based polyamine sensor with 4-mercaptobenzoic acid for

A spermine detection test was performed using the transistor-based polyamine sensor prepared in Production Example 2. The detection test was performed by immersing the detection electrode of the transistor-based polyamine sensor prepared in Production Example 2 in a liquid sample, which is a measurement solution, allowing it to stand at 25°C for 10 minutes, and then measuring the transmission characteristics of the organic thin film transistor electrically connected to the detection electrode with a semiconductor parameter analyzer. The measurement conditions were such that the gate voltage (V_{GS}) = -3.0 to 0.5 V, and the drain voltage (V_{DS}) = -1.0 V. The liquid sample used here was obtained by adding spermine (0 to 150 µM) to a 50 mM CHES buffer solution (containing 10 mM NaCl) at a pH of 8.5.

Fig. 11 is a graph showing the relationship between the gate voltage (V_{GS}) and the absolute value of drain current (I_{DS}) associated with the addition of spermine (dashed line: before addition, solid line: after addition of spermine (150 µM)). The graph shown in Fig. 11 confirmed a change in the transmission characteristics of the organic thin film transistor associated with the addition of spermine, indicating that spermine detection was possible. The positive shift of the gate voltage is assumed to be a response caused by spermine extracting the copper ion coordinately bonded to 4-mercaptobenzoic acid in the self-assembled monolayer, associated with the addition of spermine. The response reached saturation in about 5 minutes, and the response was confirmed to be stable thereafter.

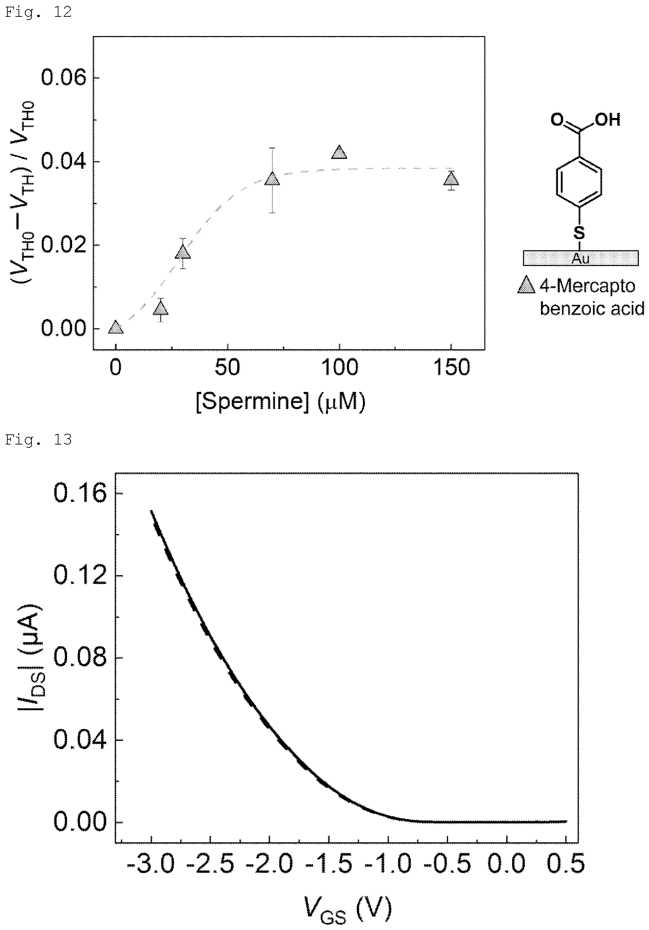

Fig. 12 is a graph showing the relationship between the amount of change in the threshold voltage and the spermine concentration. The graph shown in Fig. 12 confirmed a change in the threshold voltage associated with a change in the spermine concentration, indicating that detection of a change in the spermine concentration was possible. The response reached saturation at 70 µM, indicating that spermine detection was possible at a low concentration.

### Example 5: Spermine detection test using transistor-based polyamine sensor with 5-carboxy-1-pentanethiol

A spermine detection test was performed using the transistor-based polyamine sensor prepared in Production Example 3. The detection test was performed by immersing the detection electrode of the transistor-based polyamine sensor prepared in Production Example 3 in a liquid sample, which is a measurement solution, allowing it to stand at 25°C for 10 minutes, and then measuring the transmission characteristics of the organic thin film transistor electrically connected to the detection electrode with a semiconductor parameter analyzer. The measurement conditions were such that the gate voltage (V_{GS}) = -3.0 to 0.5 V, and the drain voltage (V_{DS}) = -1.0 V. The liquid sample used here was obtained by adding spermine (0 to 150 µM) to a 50 mM CHES buffer solution (containing 10 mM NaCl) at a pH of 8.5.

Fig. 13 is a graph showing the relationship between the gate voltage (V_{GS}) and the absolute value of drain current (I_{DS}) associated with the addition of spermine (dashed line: before addition, solid line: after addition of spermine (150 µM)). The graph shown in Fig. 13 confirmed a change in the transmission characteristics of the organic thin film transistor associated with the addition of spermine, indicating that spermine detection was possible. The positive shift of the gate voltage is assumed to be a response caused by spermine extracting the copper ion coordinately bonded to 5-carboxy-1-pentanethiol in the self-assembled monolayer, associated with the addition of spermine. The response reached saturation in about 5 minutes, and the response was confirmed to be stable thereafter.

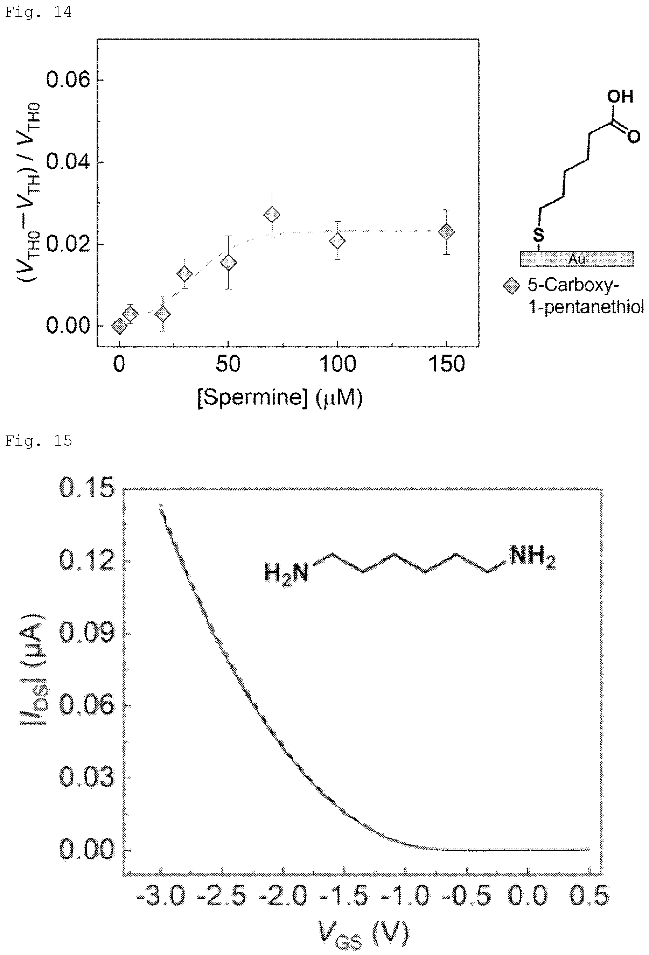

Fig. 14 is a graph showing the relationship between the amount of change in the threshold voltage and the spermine concentration. The graph shown in Fig. 14 confirmed a change in the threshold voltage associated with a change in the spermine concentration, indicating that detection of a change in the spermine concentration was possible. The response reached saturation at 50 µM, indicating that spermine detection was possible at a low concentration.

### Example 6: 1,6-Diaminohexane detection test using transistor-based polyamine sensor with TMT

A 1,6-diaminohexane detection test was performed using the transistor-based polyamine sensor prepared in Production Example 1. The detection test was performed by immersing the detection electrode of the transistor-based polyamine sensor prepared in Production Example 1 in a liquid sample, which is a measurement solution, allowing it to stand at 25°C for 10 minutes, and then measuring the transmission characteristics of the organic thin film transistor electrically connected to the detection electrode with a semiconductor parameter analyzer. The measurement conditions were such that the gate voltage (V_{GS}) = - 3.0 to 0.5 V, and the drain voltage (V_{DS}) = -1.0 V. The liquid sample used here was obtained by adding 1,6-diaminohexane (0 to 150 µM) to a 50 mM CHES buffer solution (containing 10 mM NaCl) at a pH of 8.5.

Fig. 15 is a graph showing the relationship between the gate voltage (V_{GS}) and the absolute value of drain current (I_{DS}) associated with the addition of 1,6-diaminohexane (dashed line: before addition, solid line: after addition of 1,6-diaminohexane (150 µM)). The graph shown in Fig. 15 confirmed a change in the transmission characteristics of the organic thin film transistor associated with the addition of 1,6-diaminohexane, indicating that 1,6-diaminohexane detection was possible. The negative shift of the gate voltage is assumed to be a response caused by the deposition of 1,6-diaminohexane on the complex formed by bonding of a divalent copper ion to the carboxy group constituting 2-thioacetic acid-5-mercapto-1,3,4-thiadiazole (TMT), associated with the addition of 1,6-diaminohexane. The response reached saturation in about 5 minutes, and the response was confirmed to be stable thereafter.

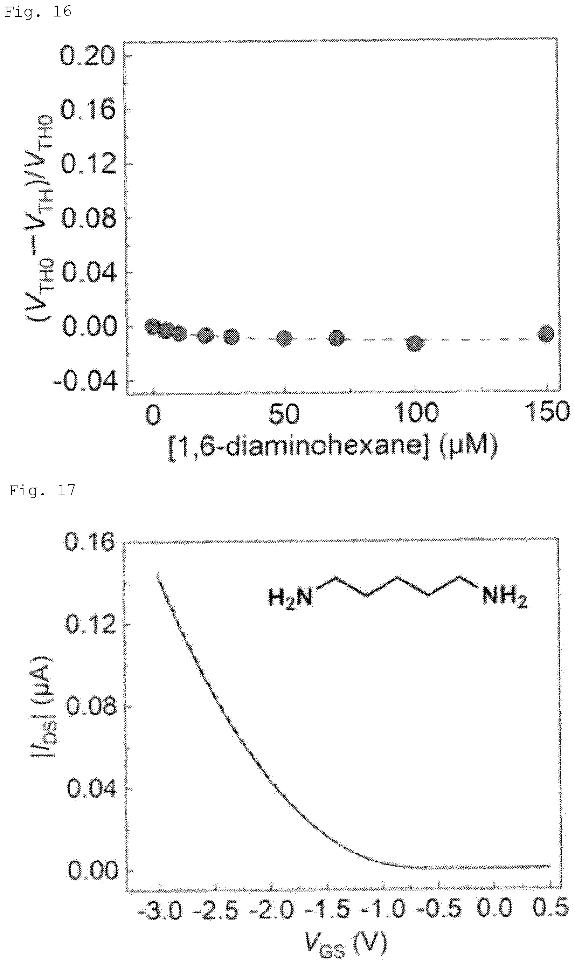

Fig. 16 is a graph showing the relationship between the amount of change in the threshold voltage and the 1,6-diaminohexane concentration. The graph shown in Fig. 16 confirmed a change in the threshold voltage associated with a change in the 1,6-diaminohexane concentration, indicating that detection of a change in the 1,6-diaminohexane concentration was possible. The response reached saturation at 50 µM, indicating that 1,6-diaminohexane detection was possible at a low concentration.

### Example 7: Cadaverine detection test using transistor-based polyamine sensor with TMT

A cadaverine detection test was performed using the transistor-based polyamine sensor prepared in Production Example 1. The detection test was performed by immersing the detection electrode of the transistor-based polyamine sensor prepared in Production Example 1 in a liquid sample, which is a measurement solution, allowing it to stand at 25°C for 10 minutes, and then measuring the transmission characteristics of the organic thin film transistor electrically connected to the detection electrode with a semiconductor parameter analyzer. The measurement conditions were such that the gate voltage (V_{GS}) = -3.0 to 0.5 V, and the drain voltage (V_{DS}) = -1.0 V. The liquid sample used here was obtained by adding cadaverine (0 to 150 µM) to a 50 mM CHES buffer solution (containing 10 mM NaCl) at a pH of 8.5.

Fig. 17 is a graph showing the relationship between the gate voltage (V_{GS}) and the absolute value of drain current (I_{DS}) associated with the addition of cadaverine (dashed line: before addition, solid line: after addition of cadaverine (150 µM)). The graph shown in Fig. 17 confirmed a change in the transmission characteristics of the organic thin film transistor associated with the addition of cadaverine, indicating that cadaverine detection was possible. The negative shift of the gate voltage is assumed to be a response caused by the deposition of cadaverine on the complex formed by bonding of a divalent copper ion to the carboxy group constituting 2-thioacetic acid-5-mercapto-1,3,4-thiadiazole (TMT), associated with the addition of cadaverine. The response reached saturation in about 5 minutes, and the response was confirmed to be stable thereafter.

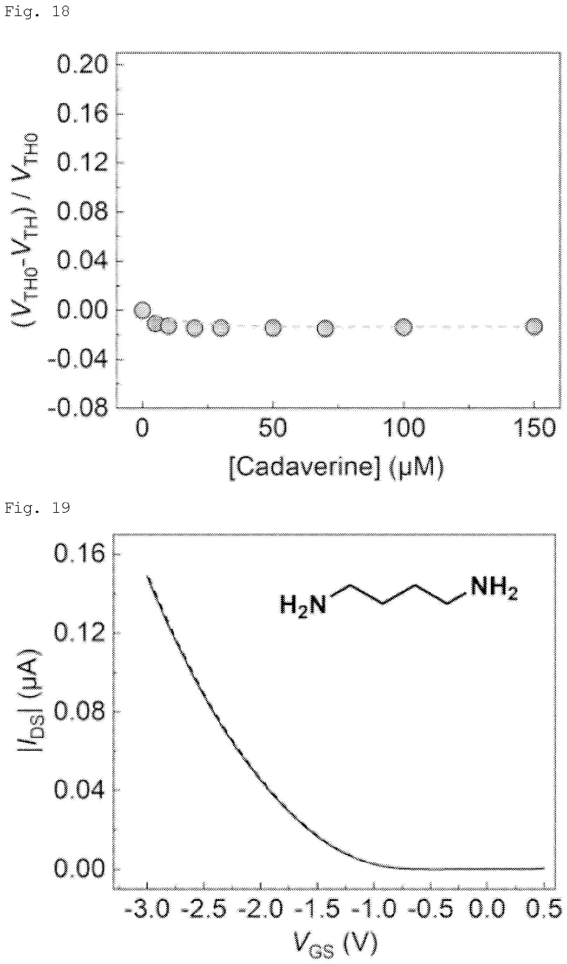

Fig. 18 is a graph showing the relationship between the amount of change in the threshold voltage and the cadaverine concentration. The graph shown in Fig. 18 confirmed a change in the threshold voltage associated with a change in the cadaverine concentration, indicating that detection of a change in the cadaverine concentration was possible. The response reached saturation at 20 µM, indicating that cadaverine detection was possible at a low concentration.

### Example 8: Putrescine detection test using transistor-based polyamine sensor with TMT

A putrescine detection test was performed using the transistor-based polyamine sensor prepared in Production Example 1. The detection test was performed by immersing the detection electrode of the transistor-based polyamine sensor prepared in Production Example 1 in a liquid sample, which is a measurement solution, allowing it to stand at 25°C for 10 minutes, and then measuring the transmission characteristics of the organic thin film transistor electrically connected to the detection electrode with a semiconductor parameter analyzer. The measurement conditions were such that the gate voltage (V_{GS}) = -3.0 to 0.5 V, and the drain voltage (V_{DS}) = -1.0 V. The liquid sample used here was obtained by adding putrescine (0 to 150 µM) to a 50 mM CHES buffer solution (containing 10 mM NaCl) at a pH of 8.5.

Fig. 19 is a graph showing the relationship between the gate voltage (V_{GS}) and the absolute value of drain current (I_{DS}) associated with the addition of putrescine (dashed line: before addition, solid line: after addition of putrescine (150 µM)). The graph shown in Fig. 19 confirmed a change in the transmission characteristics of the organic thin film transistor associated with the addition of putrescine, indicating that putrescine detection was possible. The negative shift of the gate voltage is assumed to be a response caused by the deposition of putrescine on the complex formed by bonding of a divalent copper ion to the carboxy group constituting 2-thioacetic acid-5-mercapto-1,3,4-thiadiazole (TMT), associated with the addition of putrescine. The response reached saturation in about 5 minutes, and the response was confirmed to be stable thereafter.

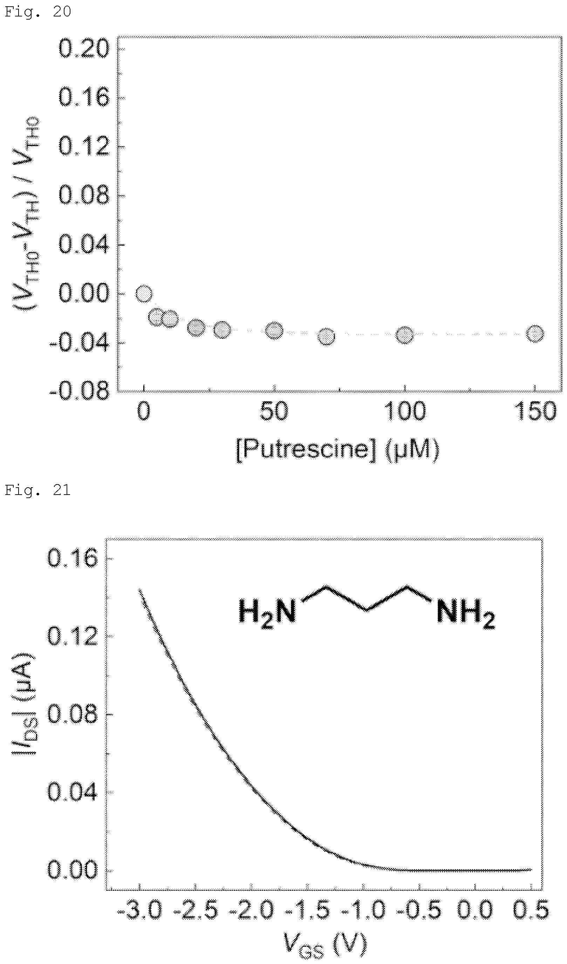

Fig. 20 is a graph showing the relationship between the amount of change in the threshold voltage and the putrescine concentration. The graph shown in Fig. 20 confirmed a change in the threshold voltage associated with a change in the putrescine concentration, indicating that detection of a change in the putrescine concentration was possible. The response reached saturation at 50 µM, indicating that putrescine detection was possible at a low concentration.

### Example 9: Trimethylenediamine detection test using transistor-based polyamine sensor with TMT

A trimethylenediamine detection test was performed using the transistor-based polyamine sensor prepared in Production Example 1. The detection test was performed by immersing the detection electrode of the transistor-based polyamine sensor prepared in Production Example 1 in a liquid sample, which is a measurement solution, allowing it to stand at 25°C for 10 minutes, and then measuring the transmission characteristics of the organic thin film transistor electrically connected to the detection electrode with a semiconductor parameter analyzer. The measurement conditions were such that the gate voltage (V_{GS}) = -3.0 to 0.5 V, and the drain voltage (V_{DS}) = -1.0 V. The liquid sample used here was obtained by adding trimethylenediamine (0 to 150 µM) to a 50 mM CHES buffer solution (containing 10 mM NaCl) at a pH of 8.5.

Fig. 21 is a graph showing the relationship between the gate voltage (V_{GS}) and the absolute value of drain current (I_{DS}) associated with the addition of trimethylenediamine (dashed line: before addition, solid line: after addition of trimethylenediamine (150 µM)). The graph shown in Fig. 21 confirmed a change in the transmission characteristics of the organic thin film transistor associated with the addition of trimethylenediamine, indicating that trimethylenediamine detection was possible. The positive shift of the gate voltage is assumed to be a response caused by trimethylenediamine extracting the copper ion coordinately bonded to 2-thioacetic acid-5-mercapto-1,3,4-thiadiazole (TMT) in the self-assembled monolayer, associated with the addition of trimethylenediamine. The response reached saturation in about 5 minutes, and the response was confirmed to be stable thereafter.

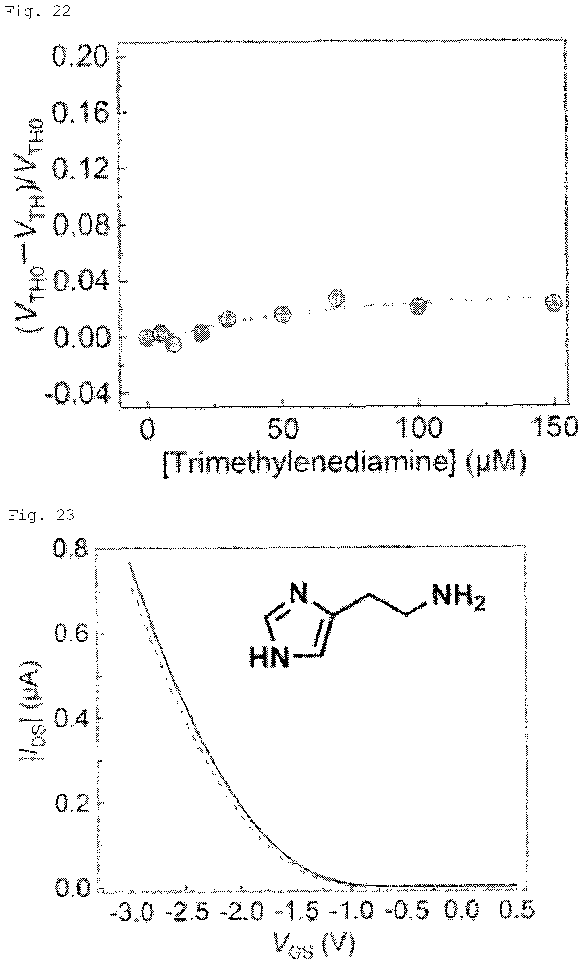

Fig. 22 is a graph showing the relationship between the amount of change in the threshold voltage and the trimethylenediamine concentration. The graph shown in Fig. 22 confirmed a change in the threshold voltage associated with a change in the trimethylenediamine concentration, indicating that detection of a change in the trimethylenediamine concentration was possible. The response reached saturation at 50 µM, indicating that trimethylenediamine detection was possible at a low concentration.

### Example 10: Histamine detection test using transistor-based polyamine sensor with TMT

A histamine detection test was performed using the transistor-based polyamine sensor prepared in Production Example 1. The detection test was performed by immersing the detection electrode of the transistor-based polyamine sensor prepared in Production Example 1 in a liquid sample, which is a measurement solution, allowing it to stand at 25°C for 10 minutes, and then measuring the transmission characteristics of the organic thin film transistor electrically connected to the detection electrode with a semiconductor parameter analyzer. The measurement conditions were such that the gate voltage (V_{GS}) = -3.0 to 0.5 V, and the drain voltage (V_{DS}) = -1.0 V. The liquid sample used here was obtained by adding histamine (0 to 150 µM) to a 50 mM CHES buffer solution (containing 10 mM NaCl) at a pH of 8.5.

Fig. 23 is a graph showing the relationship between the gate voltage (V_{GS}) and the absolute value of drain current (I_{DS}) associated with the addition of histamine (dashed line: before addition, solid line: after addition of histamine (150 µM)). The graph shown in Fig. 23 confirmed a change in the transmission characteristics of the organic thin film transistor associated with the addition of histamine, indicating that histamine detection was possible. The positive shift of the gate voltage is assumed to be a response caused by histamine extracting the copper ion coordinately bonded to 2-thioacetic acid-5-mercapto-1,3,4-thiadiazole (TMT) in the self-assembled monolayer, associated with the addition of histamine. The response reached saturation in about 5 minutes, and the response was confirmed to be stable thereafter.

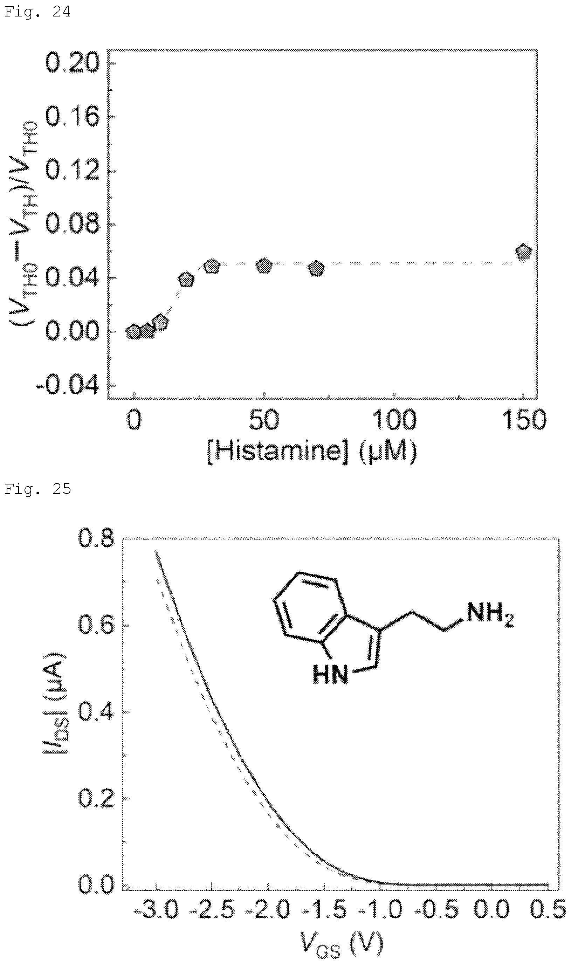

Fig. 24 is a graph showing the relationship between the amount of change in the threshold voltage and the histamine concentration. The graph shown in Fig. 24 confirmed a change in the threshold voltage associated with a change in the histamine concentration, indicating that detection of a change in the histamine concentration was possible. The response reached saturation at 30 µM, indicating that histamine detection was possible at a low concentration.

### Example 11: Tryptamine detection test using transistor-based polyamine sensor with TMT

A tryptamine detection test was performed using the transistor-based polyamine sensor prepared in Production Example 1. The detection test was performed by immersing the detection electrode of the transistor-based polyamine sensor prepared in Production Example 1 in a liquid sample, which is a measurement solution, allowing it to stand at 25°C for 10 minutes, and then measuring the transmission characteristics of the organic thin film transistor electrically connected to the detection electrode with a semiconductor parameter analyzer. The measurement conditions were such that the gate voltage (V_{GS}) = -3.0 to 0.5 V, and the drain voltage (V_{DS}) = -1.0 V. The liquid sample used here was obtained by adding tryptamine (0 to 150 µM) to a 50 mM CHES buffer solution (containing 10 mM NaCl) at a pH of 8.5.

Fig. 25 is a graph showing the relationship between the gate voltage (V_{GS}) and the absolute value of drain current (I_{DS}) associated with the addition of tryptamine (dashed line: before addition, solid line: after addition of tryptamine (150 µM)). The graph shown in Fig. 25 confirmed a change in the transmission characteristics of the organic thin film transistor associated with the addition of tryptamine, indicating that tryptamine detection was possible. The positive shift of the gate voltage is assumed to be a response caused by tryptamine extracting the copper ion coordinately bonded to 2-thioacetic acid-5-mercapto-1,3,4-thiadiazole (TMT) in the self-assembled monolayer, associated with the addition of tryptamine. The response reached saturation in about 5 minutes, and the response was confirmed to be stable thereafter.

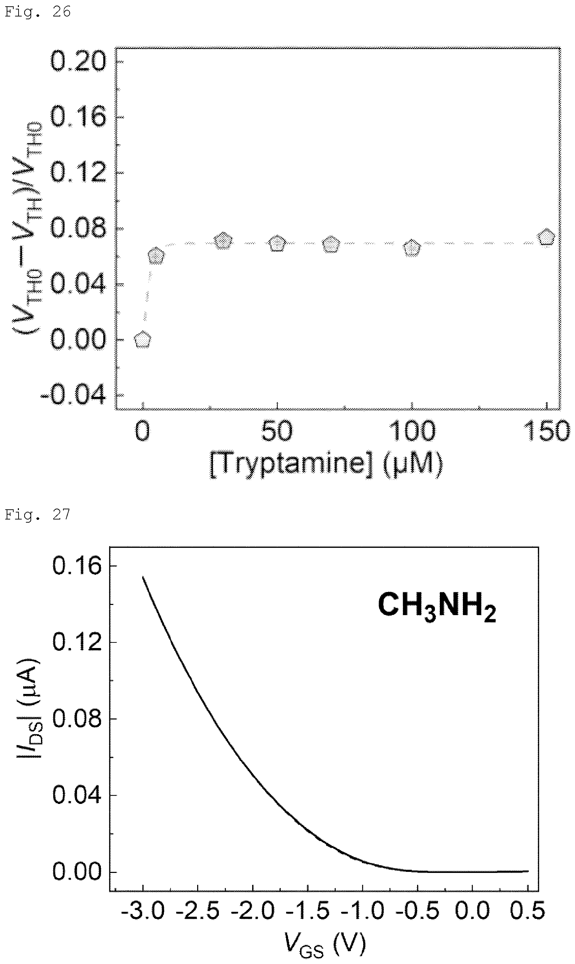

Fig. 26 is a graph showing the relationship between the amount of change in the threshold voltage and the tryptamine concentration. The graph shown in Fig. 26 confirmed a change in the threshold voltage associated with a change in the tryptamine concentration, indicating that detection of a change in the tryptamine concentration was possible. The response reached saturation at 10 µM, indicating that tryptamine detection was possible at a low concentration.

### Comparative Example 1: Methylamine detection test using transistor-based polyamine sensor with TMT

A methylamine detection test was performed using the transistor-based polyamine sensor prepared in Production Example 1. The detection test was performed by immersing the detection electrode of the transistor-based polyamine sensor prepared in Production Example 1 in a liquid sample, which is a measurement solution, allowing it to stand at 25°C for 10 minutes, and then measuring the transmission characteristics of the organic thin film transistor electrically connected to the detection electrode with a semiconductor parameter analyzer. The measurement conditions were such that the gate voltage (V_{GS}) = -3.0 to 0.5 V, and the drain voltage (V_{DS}) = -1.0 V. The liquid sample used here was obtained by adding methylamine (0 to 150 µM) to a 50 mM CHES buffer solution (containing 10 mM NaCl) at a pH of 8.5.

Fig. 27 is a graph showing the relationship between the gate voltage (V_{GS}) and the absolute value of drain current (I_{DS}) associated with the addition of methylamine (dashed line: before addition, solid line: after addition of methylamine (150 µM)). From the graph shown in Fig. 27, no positive or negative shift of the gate voltage was observed, and no change in the transmission characteristics of the organic thin film transistor associated with the addition of methylamine was observed. The results indicate that the transistor-based polyamine sensor of the present invention was unable to detect methylamine.

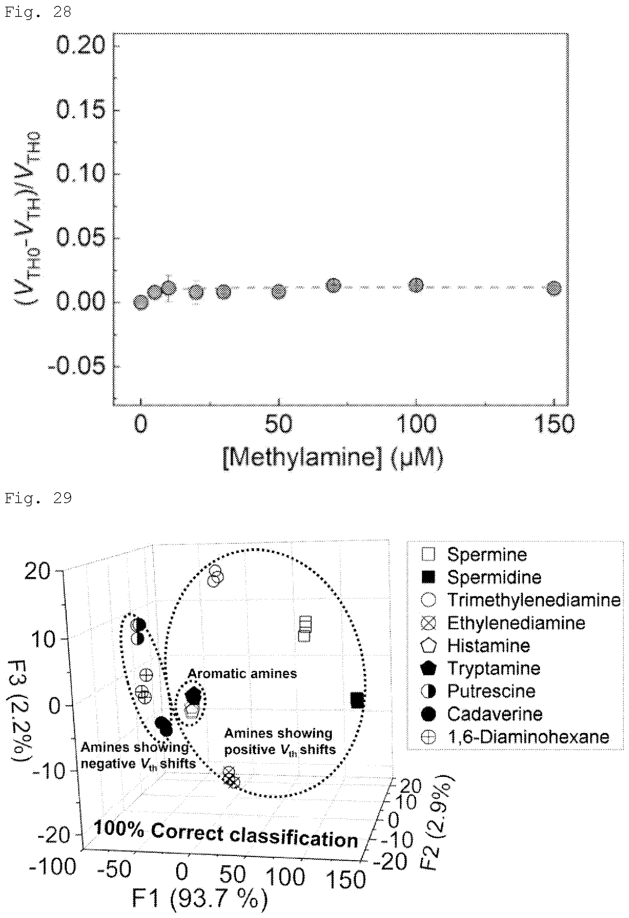

Fig. 28 is a graph showing the relationship between the amount of change in the threshold voltage and the methylamine concentration. From the graph shown in Fig. 28, a change in the threshold voltage associated with a change in the methylamine concentration could not be confirmed. The results indicate that the transistor-based polyamine sensor of the present invention was unable to detect methylamine.

### Example 12: Simultaneous identification of nine polyamines using transistor-based polyamine sensor with immobilized TMT

To evaluate the ability of the transistor-based polyamine sensor with immobilized TMT (in the Examples, simply referred to as "the polyamine sensor") for detecting multiple components, qualitative analysis of nine polyamines (ethylenediamine, spermine, spermidine, 1,6-diaminohexane, cadaverine, putrescine, trimethylenediamine, histamine, and tryptamine) was performed.

The data matrix for multicomponent analysis includes changes in transmission characteristics of the polyamine sensor obtained by adding each of the nine target polyamines (150 µM) . To obtain the data, the transmission characteristics of the polyamine sensor were measured before and after the addition of the nine target polyamines.

The gate voltage (V_{GS}) was set to -3.0 to 0.5 V. Inset data were created by using values obtained by standardizing the drain current values measured after the addition of the nine target polyamines (I_{DS}) by the drain current values before the addition (I_{DS0}). The measurement was performed three times each for the nine polyamines. The drain current values before and after the addition of each of the nine target polyamines (I_{DS} and I_{DS0}) are shown below. The transmission characteristics of the polyamine sensor were measured in terms of a single polyamine as a target species under conditions free of an impurity. (For example, after histamine was added to a buffer solution, and the transmission characteristics of the polyamine sensor was measured, tryptamine was added to a new buffer solution, and so on and so forth; in this manner, the transmission characteristics of the polyamine sensor were measured in each independent experimental system.)
(Histamine: I_{DS} = 1.4×10⁻⁹ to 7.7×10⁻⁷ A, I_{DS0} = 1.4×10⁻⁹ to 7.2×10⁻⁷ A;
Tryptamine: I_{DS} = 1.4×10⁻⁹ to 7.7×10⁻⁷ A, I_{DS0} = 1.4×10⁻⁹ to 7.2×10⁻⁷ A;
Spermine: I_{DS} = 1.9×10⁻¹⁰ to 1.6×10⁻⁷ A, I_{DS0} = 1.7×10⁻¹⁰ to 1.3×10⁻⁷ A;
Spermidine: I_{DS} = 3.3×10⁻¹¹ to 1.2×10⁻⁷ A, I_{DS0} = 3.0×10⁻¹¹ to 1.1×10⁻⁷ A;
Ethylenediamine: I_{DS} = 1.6×10⁻¹⁰ to 1.7×10⁻⁷ A, I_{DS0} = 1.3×10⁻¹⁰ to 1.4×10⁻⁷ A;
Trimethylenediamine: I_{DS} = 1.2×10⁻¹⁰ to 1.4×10⁻⁷ A, I_{DS0} = 1.1×10⁻¹⁰ to 1.4×10⁻⁷ A;
Cadaverine: I_{DS} = 1.0×10⁻¹⁰ to 1.4×10⁻⁷ A, I_{DS0} = 1.0×10⁻¹⁰ to 1.4×10⁻⁷ A;
Putrescine: I_{DS} = 1.1×0⁻¹⁰ to 1.5×10⁻⁷ A, I_{DS0} = 1.1×10⁻¹⁰ to 1.5×10⁻⁷ A;
1,6-Diaminohexane: I_{DS} = 1.1×10⁻¹⁰ to 1.4×10⁻⁷ A, I_{DS0} = 1.1×10⁻¹⁰ to 1.4×10⁻⁷ A)

Linear discriminant analysis (LDA), a type of pattern learning, was used to identify the nine target polyamines. A jackknife method (leave-one-out) was used for cross-validation. Pattern recognition can be broadly classified into supervised and unsupervised learning. Of these, LDA is categorized as supervised learning, which analyzes data while performing cross-validation.

Table 1 and Fig. 29 show the results of identification of the nine polyamines by LDA combined with a jackknife method.

Table 1 suggests that the nine target polyamines were identified with an accuracy of 100% in three repeated measurements.

**Table 1**

| | Trimethylenediamine | Putrescine | Cadaverine | 1,6-Diaminohexane | Ethylenediamine | Histamine | Spermine | Spermidine | Tryptamine | Accuracy (%) |
|---|---|---|---|---|---|---|---|---|---|---|
| Trimethylenediamine | 3 | 0 | 0 | 0 | 0 | 0 | 0 | 0 | 0 | 100 |
| Putrescine | 0 | 3 | 0 | 0 | 0 | 0 | 0 | 0 | 0 | 100 |
| Cadaverine | 0 | 0 | 3 | 0 | 0 | 0 | 0 | 0 | 0 | 100 |
| 1,6-diaminohexane | 0 | 0 | 0 | 3 | 0 | 0 | 0 | 0 | 0 | 100 |
| Ethylenediamine | 0 | 0 | 0 | 0 | 3 | 0 | 0 | 0 | 0 | 100 |
| Histamine | 0 | 0 | 0 | 0 | 0 | 3 | 0 | 0 | 0 | 100 |
| Spermine | 0 | 0 | 0 | 0 | 0 | 0 | 3 | 0 | 0 | 100 |
| Spermidine | 0 | 0 | 0 | 0 | 0 | 0 | 0 | 3 | 0 | 100 |
| Tryptamine | 0 | 0 | 0 | 0 | 0 | 0 | 0 | 0 | 3 | 100 |
| Total | 3 | 3 | 3 | 3 | 3 | 3 | 3 | 3 | 3 | 100 |

Each cluster shown in Fig. 29 is composed of three plots representing three repeated measurements. F1, F2, and F3 are the top three factors with the highest contribution in identifying the nine target polyamines, and the values in parentheses indicate the cumulative contribution of the total variance in LDA.

It was clarified that the plots identified without a single mistake formed clusters with a little variation, and that the position of the classified clusters corresponded to a change in the threshold voltage of the polyamine sensor in terms of each polyamine. In particular, the clusters of histamine and tryptamine were in close proximity to each other, the results of which clarified a high identification ability of the sensor; i.e., the sensor classified the nine target polyamines based on similarities in chemical information.

### Example 13: Quantitative detection test for spermine in mango juice (Sembikiya-Sohonten, Ltd., 100% juice (Alphonso mango))

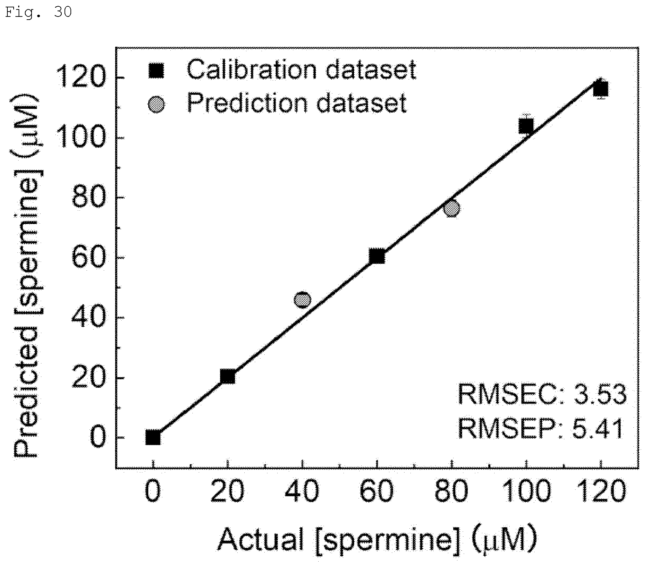

A quantitative detection test for spermine in mango juice in which spermine and polyamines other than spermine were present was performed using the transistor-based polyamine sensor prepared in Production Example 1 above. A support vector machine (SVM), a type of machine learning model, was used to predict the concentrations of spermine in the mango juice. The root mean square error (RMSE) obtained in a regression analysis can be used as an index to evaluate the accuracy of the created model. RMSEC represents the calibration curve. RMSEP represents the accuracy of the model relative to the prediction; this value decreases as the actual measurement value and predicted value become closer. The detection test was performed by immersing the detection electrode of the transistor-based polyamine sensor prepared in Production Example 1 in a liquid sample, which is a measurement solution, allowing it to stand at 25°C for 10 minutes, and then measuring the transmission characteristics of the organic thin film transistor electrically connected to the detection electrode with a semiconductor parameter analyzer. The measurement conditions were such that the gate voltage (V_{GS}) = -3.0 to 0.5 V, and the drain voltage (V_{DS}) = -1.0 V. The liquid sample used here was obtained by adding spermine to a solution in which mango juice was diluted in a 50 mM CHES buffer solution (containing 10 mM NaCl). A calibration curve was created based on data of 5 points (0, 20, 60, 100, and 120 µM), and the calibration curve was evaluated by using two points as prediction data. The results confirmed that the RMSEP was 5.41%, indicating that prediction of the spermine concentrations of 40 µM and 80 µM was achieved (Fig. 30). The horizontal axis of the graph shown in Fig. 30 represents the concentration of spermine in the liquid sample, and the vertical axis represents the concentration of spermine predicted by SVM. In Fig. 30, ■ represents a calibration plot, and ● represents a predicted value plot of spermine at unknown concentration.

### Example 14: Quantitative detection test for spermidine in mango juice (Sembikiya-Sohonten, Ltd., 100% juice (Alphonso mango))

The concentration was measured in the same manner as in Example 13, except that the measurement target was changed to spermidine, and the prediction of the spermidine concentrations was achieved.

It was confirmed that the use of the method for measuring the polyamine concentration of the present invention enables the measurement of the concentration of a polyamine to be measured even in the presence of an impurity, which is not the polyamine to be measured.

### Description of the Reference Numerals

1 Transistor site
2 Detection site
3 Sample droplet
11 Glass substrate
12 Gate electrode
13 Gate insulating film
14 Source electrode
15 Drain electrode
16 Liquid-repellent bank
17 Polymer semiconductor layer
18 Sealing film
21 PEN film substrate
22 Reference electrode

## Claims

1. A transistor-based polyamine sensor comprising a detection site, wherein
an organic compound containing a carboxy group and a thiol group is immobilized on all or part of the surface of the detection site via the thiol group of the organic compound;
a heavy metal ion is bound to the carboxy group of the organic compound; and
a change in threshold voltage caused by bonding of the heavy metal ion to a polyamine is measured to detect the polyamine.

2. The transistor-based polyamine sensor according to claim 1, wherein the organic compound has a monocyclic heterocycle containing at least one member selected from the group consisting of a nitrogen atom, a sulfur atom, and an oxygen atom.

3. The transistor-based polyamine sensor according to claim 1 or 2, wherein the organic compound is an organic compound represented by the following formula (1): 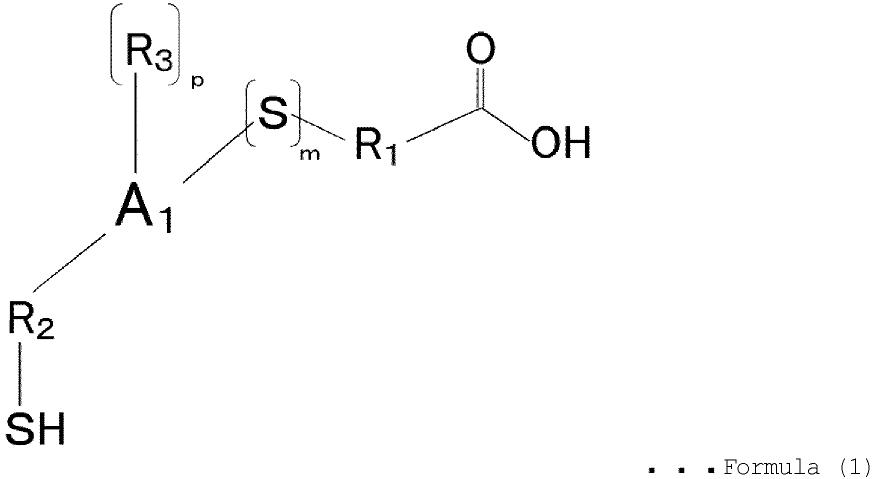 wherein
A₁ represents an aromatic hydrocarbon group, an aliphatic hydrocarbon group having 1 to 10 carbon atoms, or a monocyclic heterocyclic group containing at least one member selected from the group consisting of a nitrogen atom, a sulfur atom, and an oxygen atom;
R₁ represents an optionally substituted alkylene group having 1 to 10 carbon atoms,
R₂ represents a single bond or an optionally substituted alkylene group having 1 to 10 carbon atoms;
p is 0 or 1;
when p is 1, R₃ represents a hydrogen atom or an alkyl group having 1 to 10 carbon atoms; and
m is 0 or 1.

4. The transistor-based polyamine sensor according to claim 3, wherein the monocyclic heterocyclic group represented by A₁ in the formula (1) is a 5-membered or 6-membered monocyclic heterocyclic group containing 1 or 2 nitrogen atoms.

5. The transistor-based polyamine sensor according to claim 3 or 4, wherein the monocyclic heterocyclic group represented by A₁ in the formula (1) is a 5-membered monocyclic aromatic heterocyclic group containing 2 nitrogen atoms.

6. The transistor-based polyamine sensor according to any one of claims 3 to 5, wherein the monocyclic heterocyclic group represented by A₁ in the formula (1) is thiadiazole.

7. The transistor-based polyamine sensor according to any one of claims 3 to 6, wherein R₃ in the formula (1) is a hydrogen atom.

8. The transistor-based polyamine sensor according to any one of claims 1 to 7, wherein the polyamine is at least one member selected from the group consisting of ethylenediamine, spermine, spermidine, 1,6-diaminohexane, cadaverine, putrescine, trimethylenediamine, histamine, and tryptamine.

9. The transistor-based polyamine sensor according to any one of claims 1 to 8, wherein the heavy metal ion is at least one member selected from the group consisting of a divalent iron ion, a divalent cobalt ion, a divalent zinc ion, a divalent cadmium ion, a divalent lead ion, a divalent copper ion, and a divalent nickel ion.

10. The transistor-based polyamine sensor according to any one of claims 1 to 9, wherein the transistor is an organic thinfilm transistor.

11. A polyamine detection method comprising bringing the transistor-based polyamine sensor of any one of claims 1 to 10 into contact with a sample containing a polyamine.

12. Use of an organic compound containing a carboxy group and a thiol group as a transistor-based polyamine sensor.

13. A method for identifying a polyamine, comprising
creating a model that has undergone pattern learning by linear discriminant analysis, based on data on a change in transmission characteristics of a transistor-based polyamine sensor, the data being obtained by using the polyamine detection method of claim 11; and
identifying the polyamine by pattern recognition.

14. A method for measuring the concentration of a polyamine, comprising
measuring the concentration of a polyamine to be measured in the presence of an impurity other than the polyamine to be measured, by using a support vector machine, based on data on a change in transmission characteristics of a transistor-based polyamine sensor, the data being obtained by using the polyamine detection method of claim 11.
